# EUROPEAN PATENT SPECIFICATION

(11) **EP 4 093 427 B1**
(45) Date of publication and mention of the grant of the patent: **09.10.2024**
(21) Application number: 20717600.9
(22) Date of filing: 02.04.2020
(51) Int. Cl.: A61K 38/22, A61P 9/00

(54) **PRO-ANP FOR USE IN THE TREATMENT OF CARDIAC REMODELLING**
PRO-ANP ZUR VERWENDUNG BEI DER BEHANDLUNG VON HERZREMODELLIERUNG
UTILISATION DE PRO-ANP POUR LE TRAITEMENT DU REMODELAGE CARDIAQUE

(30) Priority: 22.01.2020 GB 202000945
(43) Date of publication of application: 30.11.2022
(73) Proprietor: Universitetet I Oslo, 0316 Oslo (NO); Oslo Universitetssykehus HF, 0424 Oslo (NO); Mahan, Lawrence, Bethesda, MD 20817 (US)
(72) Inventor: CATALIOTTI, Alessandro, 1363 Høvik (NO); ALTARA, Raffaele, 0368 Oslo (NO); MAHAN, Lawrence, Bethesda, Maryland 20817 (US)
(74) Representative: Dehns
(86) International application number: PCT/EP2020/059401
(87) International publication number: WO 2021/148143

(56) References cited:
- WO-A1-2014/185952
- US-A- 5 691 310
- US-A1- 2014 274 901
- US-A1- 2015 174 201

## Description

The present disclosure provides a polypeptidic compound for use in the prevention or treatment of adverse cardiac remodelling.

### Background

Cardiac remodelling is the process whereby the size, shape and function of the heart is altered (Cohn et al., Journal of the American College of Cardiology 35(3): 569-582, 2000). Cardiac remodelling is reviewed in Azevedo et al. (Arquivos Brasileiros de Cardiologia 106(1): 62-69, 2016) and by Cohn *et al.* (supra).

A number of manifestations of cardiac remodelling are known, including ventricular hypertrophy (whereby the walls of the ventricle thicken), ventricular fibrosis (whereby excessive fibrous tissue forms in the ventricle), cardiomegaly (in which the heart is enlarged) and ventricular dilation (in which the ventricle becomes dilated, i.e. enlarged). All forms of cardiac remodelling can cause ventricular dysfunction and malignant arrhythmias, which can lead to heart failure or even sudden death (Azevedo *et al.,* supra). Heart failure (sometimes referred to as congestive heart failure) occurs when the heart is unable to pump sufficient blood around the body to meet the body's needs and is associated with severe morbidity and mortality. Increases in rates of heart failure (primarily due to aging populations) are causing a serious economic burden on Western health services.

A frequent cause of adverse cardiac remodelling is hypertension. Despite remarkable advances in drug therapies, hypertension remains a serious medical problem with increasing prevalence and morbidity. Furthermore, a significant number of individuals exhibit either poor adherence to medicines or resistant hypertension and present severe cardiac remodelling.

The effects of cardiac remodelling are generally permanent. Compared with untreated normotensive subjects, even optimally treated hypertensive individuals are at an increased risk of an adverse cardiovascular event, due to incomplete reversal of cardiac remodelling that often persists even after several years of anti-hypertensive therapy (Struthers & George, Hypertension 66(5): 927-932, 2015). Thus, strategies that prevent or reverse the complications of hypertension and directly target cardiac damage, independent of any blood pressure lowering actions, are of pressing interest for drug development. It should be noted, however, that cardiac remodelling may be caused by any event or condition that causes injury or strain to the heart, not solely hypertension. Other conditions which can cause cardiac remodelling include myocardial infarction (Ml, also known as heart attack), myocarditis, and both idiopathic dilated cardiomyopathy and hypertrophic cardiomyopathy (Cohn *et al.,* supra).

Natriuretic peptides (NPs) secreted by the heart reduce blood pressure, improve kidney function, and prevent adverse cardiac remodelling. However, in hypertension their protective actions are diminished (Belluardo et al., American Journal of Physiology - Heart and Circulatory Physiology 291(4): H1529-1535, 2006) due to reduced gene expression (Ferrari et al., Journal of Clinical Endocrinology and Metabolism 71(4): 944-951, 1990), as well as accumulation of altered molecular forms with reduced biological value (Macheret et al., Journal of the American College of Cardiology 60(16): 1558-1565, 2012). When used as therapeutic agents, the ring structured natriuretic peptides, including atrial-natriuretic-peptide (ANP)₁₋₂₈ and B-type-natriuretic-peptide (BNP)₁₋₃₂, exhibit intense hypotensive effects.

ANP is synthesised as a preprohormone, known as preproANP, a 151 amino acid polypeptide with the sequence set forth in SEQ ID NO: 2. Cleavage of the N-terminal signal sequence yields proANP, a 126 amino acid polypeptide with the sequence set forth in SEQ ID NO: 3 (also referred to as proANP₁₋₁₂₆). ProANP is stored in atrial granules. Following release from these granules, proANP is cleaved by the serine protease corin to yield the mature ANP peptide α-ANP, which constitutes the C-terminal 28 amino acids of proANP. The amino acid sequence of α-ANP is set forth in SEQ ID NO: 4. The resulting N-terminal fragment of this cleavage reaction (proANP₁₋₉₈, SEQ ID NO: 5) is subsequently cleaved into three fragments: proANP₁₋₃₀ (SEQ ID NO: 6), proANP₃₁₋₆₇ (SEQ ID NO: 1) and proANP₇₉₋₉₈ (SEQ ID NO: 7) (Potter et al., Handbook of Experimental Pharmacology 191: 341-366, 2009; De Palo et al., Clinical Chemistry 46(6): 843-847, 2000). US 2014/274901 also discloses the use of proANP₁₋₁₂₆ polypeptides (and the fragment proANP₂₁₋₁₂₆) for treating cardiorenal disease including acute decompensated heart failure.

Mature α-ANP is produced by the heart when under stress and acts to decrease blood pressure by vasodilation and by increasing natriuresis and diuresis. These activities are primarily mediated by its binding to natriuretic peptide receptor-A (NPR-A). All mature natriuretic peptides (including α-ANP) have a 17 amino acid disulphide ring, which is required for their binding to NPR-A (Potter *et al.,* supra).

ProANP₃₁₋₆₇ is a linear peptide which has a unique mode of action. ProANP₃₁₋₆₇ does not contain the 17 amino acid ring found in mature natriuretic peptides, and does not exert its functions by activating the classic natriuretic peptide receptors (NPR-A and NPR-B). It appears to be resistant to degradation by neprilysin and other endopeptidases, and is excreted largely intact in urine. ProANP₃₁₋₆₇ is known to stimulate prostaglandin E₂ (PGE₂) formation in the kidney (Chiou & Vesely, Endocrinology 136(5): 2033-2039, 1995). Renal PGE₂ acts to increase renal blood flow and glomerular filtration rate. Recombinant proANP₃₁₋₆₇ has been shown to protect against ischemia-induced acute tubular necrosis and renal failure (Clark et al., American Journal of Physiology - Heart and Circulatory Physiology 278: H1555-1564, 2000). On this basis, proANP₃₁₋₆₇ (also known as vessel dilator, VSDL) has been found to be a safe and effective treatment for renal impairment in patients with congestive heart failure (Delacroix et al., Journal of the American College of Cardiology 67(13): Abstract 1351, 2016).

ProANP₃₁₋₆₇ is also a potent vasodilator, and has been found to have beneficial effects on cardiac performance in patients with heart failure. In particular, improvements have been found in systemic vascular resistance, pulmonary vascular resistance, pulmonary capillary wedge pressure, central venous pressure, cardiac output, cardiac index and stroke volume index, in patients with moderate heart failure (Vesely et al., Circulation 98(4): 323-329, 1998). WO 2012/019237 teaches the use of proANP₃₁₋₆₇ in the treatment of heart failure, including acute decompensated heart failure (ADHF). WO 2014/138796 more broadly teaches the use of proANP₃₁₋₆₇ in treatment of cardiorenal syndromes, including heart failure, ADHF, pulmonary arterial hypertension (PAH; according to the World Health Organisation (WHO) classification, PAH is Group 1 pulmonary hypertension), acute renal failure, chronic renal failure and acute kidney injury. WO 2015/135024 teaches the use of proANP₃₁₋₆₇ in combination with a diuretic agent for the treatment of conditions in which diuresis is desired, in particular impaired kidney function or diuretic resistance in patients with cardiorenal syndrome, heart failure (including ADHF), PAH and various kidney diseases.

### Summary

The present inventors have found that proANP₃₁₋₆₇ acts to prevent cardiac remodelling induced by hypertension, even at very low dosages of proANP₃₁₋₆₇ which do not affect blood pressure or demonstrate renal protective and diuretic effects. This discovery opens up new treatment avenues using proANP₃₁₋₆₇ to prevent cardiac remodelling in patients at risk, particularly in patients for whom lower blood pressure could be deleterious.

Accordingly, in a first aspect, the present disclosure provides a polypeptidic compound for use in prevention or treatment of cardiac remodelling in a subject, wherein said polypeptidic compound comprises the amino acid sequence set forth in SEQ ID NO: 1, or an amino acid sequence having at least 90 % sequence identity thereto, and wherein said compound comprises at least 34 amino acid residues and no more than 50 amino acid residues.

### Detailed Description

The invention is defined by the appended claims.

Human preproANP (SEQ ID NO: 2) is encoded by the gene *NPPA* and has the GenBank accession number NP_006163. As described above, this is processed to yield a number of peptide fragments, one of which is proANP₃₁₋₆₇. Human proANP₃₁₋₆₇ has (i.e. consists of) the amino acid sequence set forth in SEQ ID NO: 1.

The present inventors have discovered that proANP₃₁₋₆₇ has a direct protective effect on the heart. As shown in the examples below, the peptide counteracts cardiac remodelling, preventing cardiac hypertrophy or enlargement, and preserving diastolic function. The present disclosure thus provides new therapies which prevent cardiac remodelling, preserving normal cardiac function, in patients suffering from conditions which may lead to cardiac remodelling. ProANP₃₁₋₆₇ was previously known to improve renal function and to have beneficial haemodynamic effects in patients suffering from heart failure (Vesely *et al.*, supra). Its haemodynamic effects have been attributed to its activity as a vasodilator, which causes a reduction in blood pressure and in the pressure on the chambers of the heart.

The present inventors have found that proANP₃₁₋₆₇ has cardioprotective properties even at doses which are too low to impact on blood pressure, demonstrating a direct protective effect of proANP₃₁₋₆₇ on the heart independent of its known haemodynamic effects. Furthermore, the inventors have observed cardioprotective effects of proANP₃₁₋₆₇ even at doses too low to elicit renal effects, indicating that the anti-remodelling action on the heart is also independent of the favourable effects achievable in the kidney. The disclosure thus provides new therapeutic options for prevention and treatment of cardiac remodelling.

The term "cardiac remodelling" is well known in the art, and is defined as a group of molecular, cellular and interstitial changes that manifest as changes in the size, shape and function of the heart. These changes have a negative impact on cardiac function and eventually lead to heart failure.

The most common form of cardiac remodelling is ventricular hypertrophy, in which the walls of one or other ventricle of the heart become thicker. Ventricular hypertrophy may be concentric (in which the ventricle wall is thickened without a corresponding increase in ventricular size, resulting in a reduction in ventricular volume) or eccentric (in which thickening of the ventricle wall is associated with an increase in ventricular size and volume). Ventricular hypertrophy can cause loss of elasticity in the ventricle wall, resulting in cardiac dysfunction. Hypertrophy of the left ventricle occurs as a result of excess afterload, commonly caused by hypertension. Any other condition which causes excess left-side afterload may also result in left ventricular hypertrophy, including aortic stenosis and aortic insufficiency (also known as aortic regurgitation). Right ventricular hypertrophy may be caused by any condition which causes excess right-side afterload, in particular pulmonary hypertension.

Ventricular hypertrophy may affect one or both ventricles. Thus cardiac remodelling according to the present disclosure may refer to left ventricular hypertrophy or right ventricular hypertrophy. A common form of hypertrophy is mainly localised to the left ventricle. Another form of cardiac remodelling, less common, is double ventricular hypertrophy, in which hypertrophy of both the left and right ventricles occurs concurrently. Cardiac remodelling as referred to herein includes concentric ventricular hypertrophy and eccentric ventricular hypertrophy.

Another form of cardiac remodelling is atrial enlargement (also referred to as atrial dilation). In atrial enlargement, the size (i.e. volume) of the atrium is increased. Atrial enlargement may be seen as swelling of the atrium. Atrial enlargement can occur in either the left atrium or the right atrium. Right atrial enlargement can be caused by a number of conditions, including pulmonary hypertension. Left atrial enlargement may be caused by a number of conditions, including hypertension. Atrial enlargement may cause cardiac dysfunction, including atrial fibrillation and heart failure. Thus cardiac remodelling according to the present disclosure may refer to left atrial enlargement or right atrial enlargement.

Any enlargement of the heart may be considered a form of cardiomegaly. Cardiomegaly is a form of cardiac remodelling, and is associated with an increased risk of heart failure or sudden death. Ventricular hypertrophy and atrial enlargement may both be forms of cardiomegaly. Another form of cardiomegaly is dilated cardiomyopathy, which accordingly is a form of cardiac remodelling. In dilated cardiomyopathy the walls of one or both of the ventricles become thin and stretched, resulting in ventricular enlargement. Dilated cardiomyopathy can cause an irregular heartbeat or heart failure.

Cardiomegaly generally, and specifically ventricular hypertrophy, atrial enlargement and dilated cardiomyopathy are forms of cardiac remodelling associated with changes in the size of the heart. Such changes commonly also result in a change in the shape of the heart from a generally elliptical shape to a more spherical shape. The presently disclosed method may thus be used to prevent or treat cardiomegaly, ventricular hypertrophy (including left and right ventricular hypertrophy), atrial enlargement (including left and right atrial enlargement) and dilated cardiomyopathy. In a particular embodiment, the presently disclosed method provides prevention or treatment of ventricular hypertrophy, particularly left ventricular hypertrophy.

Another form of cardiac remodelling is cardiac fibrosis. Cardiac fibrosis refers to the excess deposition of extracellular matrix on cardiac muscle, in particular excess deposition of collagen type I and III. Cardiac fibrosis results in increased tensile strength of the affected cardiac muscle, which results in stiffness or rigidity of the cardiac muscle, causing loss of cardiac function. In particular, increased stiffness of the cardiac muscle leads to a reduced ejection fraction, and may thus lead to heart failure. Excess collagen deposition is also believed to impact electrical conductivity of the heart, which may impair normal heart function. Cardiac fibrosis is a response to cardiac injury, the most common cause of which is heart attack (myocardial infarction).

Cardiac fibrosis may take place in any location in the heart. Fibrosis may occur in a ventricle (ventricular fibrosis) or an atrium (atrial fibrosis). Both ventricular fibrosis and atrial fibrosis may occur on the left or right side of the heart, i.e. cardiac fibrosis includes right ventricular fibrosis, left ventricular fibrosis, right atrial fibrosis and left atrial fibrosis.

Left ventricular hypertrophy and left ventricular fibrosis can lead to diastolic dysfunction. Both ventricular hypertrophy and ventricular fibrosis cause the ventricle wall to become stiff, preventing the ventricle from relaxing properly between beats. This essentially results in a decreased ventricle volume, resulting in a reduction in the volume of blood pumped by the heart with each beat. This condition is known as diastolic dysfunction, which may evolve to overt heart failure and is a typical manifestation of heart failure with preserved systolic function (i.e. normal ejection fraction), a severe condition suffered by an increasing number of patients, with a high mortality rate and for which there is lack of effective treatment.

Methods by which the various types of cardiac remodelling may be diagnosed are known in the art. Cardiomegaly, including ventricular hypertrophy and atrial enlargement, can be diagnosed using any procedure suitable for cardiac imaging. Particularly suitable methods include echocardiography, computerised tomography (CT) and magnetic resonance imaging (MRI). Generally, left ventricular hypertrophy is defined as a left ventricular myocardium thickness of more than 1.1 cm; right ventricular hypertrophy is defined as a right ventricular free wall thickness of more than 5 mm; left atrial enlargement is defined as a left atrium long axis dimension (diameter) of more than 38 mm (in women) or more than 40 mm (in men); right atrial enlargement is defined as a long axis dimension of the right atrium of more than 45 mm.

Cardiac fibrosis may also be diagnosed by MRI, in particular using a gadolinium-based contrast agent. Another suitable method for cardiac fibrosis diagnosis is by quantification of the interstitial collagen content of a cardiac sample obtained by endomyocardial biopsy.

The subject treated according to the present disclosure may be any animal with a heart. The subject may be any mammal. It may be any domestic, livestock or sports animal. Preferably however, the subject is a human. By "the subject treated" as used herein is meant the subject to whom the polypeptidic compound is administered according to the disclosure, to prevent or treat cardiac remodelling.

The polypeptidic compound for use according to the disclosure may be used to prevent cardiac remodelling (and thus the development of heart failure) in a healthy subject at risk of developing cardiac remodelling. By "healthy" as used herein is meant a subject who does not have heart failure, or at least does not have overt symptoms of heart failure. A healthy subject, as referred to herein, may not be absolutely healthy, i.e. a healthy subject may suffer from diseases or medical conditions which are not heart failure.

The symptoms of heart failure are well known to the skilled physician. Such symptoms include shortness of breath, fatigue/fainting, chest pain, irregular or rapid heartbeat, and swelling of the abdomen, legs, ankles and/or feet. The skilled physician is capable of identifying the symptoms of heart failure in a subject. A subject who does not have overt symptoms of heart failure is a subject in whom the symptoms of heart failure, as listed above, are not apparent. A subject who does not have overt symptoms of heart failure may nonetheless display signs of cardiac remodelling if investigated using a suitable diagnostic technique, as detailed above. A subject who does not have overt symptoms of heart failure may be considered to be a subject who does not suffer from heart failure. Thus the polypeptidic compound described herein may be used according to the disclosure to prevent cardiac remodelling, and thus prevent the development of heart failure (i.e. the onset of heart failure), in a subject not suffering from heart failure.

Thus the polypeptidic compound described herein may be used to prevent cardiac remodelling in a subject who does not suffer from chronic heart failure (also known as chronic congestive heart failure). Chronic heart failure is a long-term condition (the term "heart failure" is commonly used to refer to chronic heart failure). Such use of the polypeptidic compound described herein may prevent the development of chronic heart failure. As noted above, a subject who does not have heart failure may nonetheless display cardiac remodelling. In the context of a subject who does not have heart failure and does not display cardiac remodelling, if examined, the polypeptidic compound described herein may be used to prevent the initiation of cardiac remodelling. In the context of a subject who does not have heart failure but does display signs of cardiac remodelling, if examined, the polypeptidic compound described herein may be used to prevent the worsening of cardiac remodelling. Alternatively, in the context of a subject who does not have heart failure but does display signs of cardiac remodelling, the polypeptidic compound described herein may be used to treat (i.e. reverse) the cardiac remodelling.

The polypeptidic compound described herein may be used to treat existing cardiac remodelling, or to prevent further cardiac remodelling, in a subject who already has heart failure, thus to improve the subject's condition or to prevent worsening of the subject's heart failure. The severity of heart failure may be classified according to the New York Heart Association (NYHA) classification. This grades heart failure into four classes defined based on severity of symptoms. NYHA class I and II heart failure is mild: a subject with NYHA class I heart failure does not experience limitation in their physical activity, in that ordinary physical activity does not cause onset of symptoms of heart failure (undue fatigue, palpitations, dyspnea or chest pain); a subject with NYHA class II heart failure is comfortable at rest but experiences limitation in physical activity, in that ordinary physical activity results in symptoms of heart failure (fatigue, palpitations, dyspnea or chest pain). NYHA class III heart failure is moderate: a subject with NYHA class III heart failure is comfortable at rest, but experiences considerable limitation of physical activity in that less than ordinary activity causes symptoms of heart failure (fatigue, palpitations, dyspnea or chest pain). NYHA class IV heart failure is severe: a subject with NYHA class IV heart failure is unable to undertake any physical activity without experiencing symptoms of heart failure, and may experience such symptoms when at rest.

The polypeptidic compound according to the disclosure may be used to treat or prevent cardiac remodelling in a subject who does not have heart failure with a symptom severity level of NYHA class III or class IV. That is to say the disclosure may be used in treatment of prevention of cardiac remodelling in a subject who has no overt symptoms of heart failure, and thus does not register on the NYHA classification. In such an individual the polypeptidic compound according to the disclosure is used to prevent onset of heart failure. As discussed below, a subject treated according to the current disclosure may be a subject who has no overt symptoms of heart failure, but is at risk of developing heart failure. The disclosure may alternatively be used to treat existing cardiac remodelling, and/or prevent further cardiac remodelling, in a subject with NYHA class I or NYHA class II heart failure.

In another embodiment, the subject according to the present disclosure does not have overt symptoms of acute decompensated congestive heart failure (ADCHF; also referred to acute decompensated heart failure, ADHF). ADCHF is a sudden worsening of existing heart failure. Thus, in an embodiment, the subject has stable heart failure (i.e. heart failure in which the symptoms are largely unchanging over time) and is not in the midst of an acute decompensation episode.

In another embodiment, the subject treated according to the present disclosure does not have overt symptoms of a cardiorenal syndrome. Cardiorenal syndromes are conditions of the heart and kidneys whereby acute or chronic dysfunction in one organ induces acute or chronic dysfunction of the other. Examples of cardiorenal syndromes include chronic heart failure, ADCHF, acute kidney failure, glomerulonephritis and chronic glomerular disease. Other cardiorenal syndromes are known in the medical arts. In line with the description above, a subject who does not have overt symptoms of a cardiorenal syndrome is a subject in whom the symptoms of a cardiorenal syndrome are not apparent. In another embodiment, the subject treated according to the present disclosure does not have overt symptoms of a cardiorenal syndrome or overt symptoms of heart failure.

As noted above, cardiac remodelling can lead to heart failure. Accordingly, a subject treated according to the present disclosure may in particular be a subject at risk of heart failure. A subject "at risk" of heart failure is a subject who has an increased risk of developing heart failure relative to the population average. Such a subject may be a subject having a medical condition which predisposes the subject to developing, or causes, heart failure. A subject at risk of developing heart failure may also, or alternatively, be a subject with a genetic predisposition to heart failure.

As noted above, left ventricular hypertrophy is most commonly a result of hypertension (high blood pressure). The subject treated according to the disclosure may therefore be a subject who has hypertension. A subject is defined herein as having hypertension in line with accepted international guidelines, e.g. a subject having a high systolic blood pressure (of at least 140 mmHg) and/or a high diastolic blood pressure (of at least 90 mmHg). The hypertension may be primary hypertension (also known as essential hypertension or idiopathic hypertension, which has no identifiable cause) or secondary hypertension (hypertension having an identifiable underlying primary cause).

In another embodiment the subject treated according to the disclosure is a subject with high normal blood pressure (i.e. a subject who does not have hypertension, as defined above, but has a systolic blood pressure between 130 and 140 mmHg and/or a diastolic blood pressure between 85 and 90 mmHg), particularly such a subject who is at risk of heart failure. For example the subject may have high normal blood pressure and a cardiovascular disease, and may therefore be at high risk of heart failure. In particular, the subject may be an individual with high normal blood pressure and coronary artery disease.

In a particular embodiment the subject is a pregnant woman who has developed hypertension as a complication of her pregnancy. The subject may have gestational hypertension: hypertension which has developed during pregnancy but without any other identifiable cause. Alternatively, the subject may have pre-eclampsia, a condition with a number of possible symptoms including headache, oedema and visual problems. The skilled physician is able to distinguish between gestational hypertension and pre-eclampsia: pre-eclampsia is generally associated with proteinuria, which is not found in gestational hypertension; pre-eclampsia may also be indicated by low levels of placental growth factor (PGF) in the blood.

A subject with hypertension, or high normal blood pressure and a cardiovascular disease, may be administered a polypeptidic compound according to the disclosure in order to prevent or treat cardiac remodelling, in combination with an antihypertensive medication to reduce blood pressure. A polypeptidic compound according to the disclosure may be administered to a subject with hypertension in combination with any antihypertensive medication, e.g. an ACE inhibitor, beta-blocker, calcium channel blocker or angiotensin receptor blocker (ARB). A polypeptidic compound according to the disclosure may be administered in combination with one or more (e.g. two or three) antihypertensive medications.

In a particular embodiment, the subject treated according to the disclosure has resistant hypertension. "Resistant hypertension" is defined herein in accordance with standard medical guidelines, as hypertension which remains uncontrolled (i.e. in which the blood pressure remains above the threshold for hypertension, as defined above) despite simultaneous treatment with at least three antihypertensive drugs with different mechanisms of action. Generally, a hypertensive subject's risk of developing heart failure may be significantly reduced by antihypertensive therapy, which reduces blood pressure and thus reduces the afterload on the left ventricle. In this way, cardiac remodelling may be prevented, and existing cardiac remodelling may, at least to some extent, be reversed. A subject with resistant hypertension is at particular risk of heart failure, since the subject's blood pressure cannot be reduced to a healthy level. The uncontrolled high blood pressure results in cardiac remodelling, and leads to a greatly increased risk of heart failure.

As noted above, the polypeptidic compound for use according to the present disclosure has a direct protective effect on the heart despite the presence of high blood pressure, and thus may be of particular importance in treating adverse cardiac remodelling in subjects with resistant hypertension. This is because the polypeptidic compound may be used to prevent cardiac remodelling, reducing the risk of heart failure, which is otherwise not possible due to the failure of the subject to respond to antihypertensive therapy.

The potential of proANP₃₁₋₆₇ for use in treatment of resistant hypertension is a particularly surprising and advantageous aspect of the disclosure. As detailed above, proANP₃₁₋₆₇ is known to have beneficial haemodynamic effects, but this was thought to be due to the antihypertensive effect of proANP₃₁₋₆₇, which is a result of its vasodilatory activity. On this basis, individuals with resistant hypertension would not have been expected to benefit from proANP₃₁₋₆₇ therapy. By demonstrating that proANP₃₁₋₆₇ directly acts to protect the heart from remodelling, independent of any effect on blood pressure, the present disclosure provides an important new treatment option for a patient group for which, currently, minimal treatment options are available. The present disclosure may in particular, therefore, be used to prevent cardiac remodelling in a subject with resistant hypertension, and even to reverse existing cardiac remodelling in such a subject.

The subject treated according to the present disclosure may have any other condition which may cause cardiac remodelling, or puts the subject at risk of developing heart failure. For instance, the subject may have a heart valve disorder. Heart valve disorders frequently drive cardiac remodelling, by putting increased pressure on the relevant chamber of the heart. Heart valve disorders include aortic stenosis, in which a narrowing of the aortic valve opening restricts passage of blood from the left ventricle to the aorta, causing increased pressure on the left ventricle and thus left ventricular hypertrophy. Another heart valve disorder is aortic insufficiency (also known as aortic regurgitation), in which the aortic valve leaks, allowing blood to pass through in the reverse direction (i.e. from the aorta into the left ventricle). This leakage causes increased pressure on the left ventricle, and thus left ventricular hypertrophy. Similarly, disorders of the mitral valve (particularly mitral stenosis and mitral regurgitation) can cause left atrial enlargement, as they cause increased pressure on the left atrium; disorders of the tricuspid valve (particularly tricuspid valve stenosis and tricuspid valve regurgitation) can cause right atrial enlargement; and disorders of the pulmonary valve (particularly pulmonary valve stenosis and pulmonary valve regurgitation) can cause right ventricular hypertrophy. The present disclosure may be used to treat a subject with any such heart valve disease, to prevent and/or treat cardiac remodelling in the subject.

The subject treated according to the present disclosure may have an endocrine (hormonal) disorder. Several endocrine disorders are known to be associated with cardiac remodelling, as described below. An endocrine disorder is any disorder of the endocrine system, in particular a disorder associated with hypersecretion or hyposecretion of one or more hormones from an endocrine gland. More generally the subject may have a metabolic disorder. This may include metabolic syndrome, or insulin resistance or any condition associated with insulin resistance.

One such endocrine disorder is diabetes mellitus (diabetes). As is known to the skilled person, there are two types of diabetes: type 1 (which is an autoimmune condition in which an autoimmune response causes destruction of the insulin-producing beta cells of the pancreas); and type 2 (which is caused by insulin resistance, in which cells fail to respond properly to insulin). Both types of diabetes, but particularly type 2, are associated with an increased risk of developing heart failure, indicating that diabetes promotes cardiac remodelling. The subject treated according to the disclosure may thus be a subject with diabetes. In particular, the subject treated according to the disclosure may have type 2 diabetes.

Obesity is a significant risk factor for both hypertension and diabetes, meaning obese individuals are at particular risk of heart failure. The present disclosure may thus be used to treat or prevent cardiac remodelling in an obese subject.

Another endocrine disorder associated with cardiac remodelling is thyroid disease. Both hypothyroidism and hyperthyroidism are associated with cardiac remodelling and heart failure. Thus the subject may have hypothyroidism or hyperthyroidism. Parathyroid disease is also associated with cardiac remodelling: hyperparathyroidism is associated with hypertension and obesity; hypoparathyroidism is associated with decreased cardiac performance, and increased risk of developing dilated cardiomyopathy. Thus the subject treated according to the method disclosed herein may have parathyroid disease, e.g. hyperparathyroidism or hypoparathyroidism.

The subject treated according to the disclosed method may have primary aldosteronism, a condition in which excess aldosterone is produced by the adrenal glands. Primary aldosteronism may have a genetic cause (familial hyperaldosteronism), or may be caused by enlargement of the adrenal glands (adrenal hyperplasia) or adrenal adenoma or cancer. Primary aldosteronism is associated with cardiac remodelling, in that the condition causes hypertension.

The subject treated according to the disclosed method may have a pheochromocytoma, a neoplastic condition of the adrenal gland. Tumour formation results in excess production of adrenaline and noradrenaline, causing hypertension and potentially leading to cardiac remodelling, putting pheochromocytoma patients at risk of heart failure.

In another embodiment the subject treated according to the present method has growth hormone deficiency (GHD). GHD commonly has a genetic cause, though may also be caused by trauma, infections, tumours or other external factors. Generally, growth hormone deficiency arises due to problems with the pituitary gland. GHD has a number of symptoms which may cause cardiac damage, e.g. increased body fat and obesity which can cause hypertension, and insulin resistance which can cause diabetes.

In another embodiment the subject treated according to the present method has acromegaly, a pituitary gland disease in which excess growth hormone is produced, normally due to the presence of a benign tumour in the pituitary gland. Complications of acromegaly include hypertension and diabetes, which as detailed above can cause cardiac remodelling. Acromegaly can also directly cause cardiomyopathy.

In another embodiment the subject treated according to the method disclosed herein has Cushing's syndrome. Cushing's syndrome may be caused by excess cortisol production by the adrenal glands, which may be caused by a tumour in the pituitary gland causing excess adrenocorticotropic hormone production or a tumour in the adrenal gland producing excess cortisol. More commonly, Cushing's syndrome is caused by the use of prescribed glucocorticoids. Cushing's syndrome has a number of effects which may cause cardiac remodelling, including causing hypertension, obesity and diabetes.

As detailed above, another significant cause of cardiac remodelling is myocardial infarction (heart attack), which can in particular cause cardiac fibrosis. In a particular embodiment the subject treated according to the present disclosure is a subject who has suffered a myocardial infarction. A polypeptidic compound according to the disclosure may be administered to a subject who has suffered a myocardial infarction to prevent cardiac remodelling, or to prevent further cardiac remodelling and reverse cardiac remodelling which has already taken place. For maximum effect, the polypeptidic compound according to the disclosure should be administered to a subject who has suffered a myocardial infarction as soon as possible after the event, in order to minimise cardiac remodelling as a result. For instance, it is envisaged that first responders (such as paramedics) would be equipped with a polypeptidic compound according to the disclosure, to prevent delay in administration of the polypeptidic compound. Nonetheless, a subject who has suffered a myocardial infarction may usefully be administered a polypeptidic compound according to the disclosure after the event, to treat cardiac remodelling which occurred as a result.

In another embodiment, the subject treated according to the disclosure has myocarditis (inflammation of the heart muscle, generally caused by a viral infection). In another embodiment the subject treated according to the disclosure has cardiomyopathy, which is described above. Cardiomyopathy may be caused by e.g. a viral infection, hypertension, a disease of the tissue, etc. Cardiomyopathy may alternatively be a genetic condition (familial cardiomyopathy). Subjects with either familial cardiomyopathy or non-familial cardiomyopathy may be treated according to the present disclosure.

In another embodiment the subject treated according to the disclosure has amyloidosis, a condition in which amyloid deposits in the heart can lead to heart failure.

In another embodiment, the subject treated according to the disclosure has haemochromatosis, an inherited condition characterised by iron build up in the body. Buildup of iron in the heart can cause cardiac remodelling and heart failure.

In another embodiment the subject treated according to the disclosure has an arrhythmia. Arrhythmias are conditions in which the heartbeat is not properly coordinated (i.e. does not have a correct rhythm). Arrhythmia may cause a slow heartbeat (bradycardia), a fast heartbeat (tachycardia) or an irregular heartbeat (fibrillation). Arrhythmias may be caused by an array of conditions. Tachycardia and bradycardia in particular can put the heart under excessive pressure, leading to cardiac remodelling and, eventually, heart failure. Thus the subject treated according to the disclosure may have an arrhythmia. In a particular embodiment, the subject has tachycardia. In another embodiment, the subject has bradycardia.

In another embodiment, the subject treated according to the present disclosure has chronic obstructive pulmonary disease (COPD). COPD is a condition which causes breathing difficulties, primarily caused by damage to the airways as a result of smoking. A common complication of COPD is pulmonary hypertension (specifically of WHO Group III, pulmonary hypertension linked with lung disease or hypoxia), which as described above causes right ventricular hypertrophy.

In another embodiment, the subject treated according to the present disclosure has sarcoidosis. In sarcoidosis, collections of white blood cells form granulomas, which develop in organs of the body. Commonly, the lungs are affected, which can cause pulmonary hypertension (specifically of WHO Group V, pulmonary hypertension of unclear or multifactorial mechanism).

In another embodiment the subject may be a drug or alcohol user. In particular, the subject may be abusing alcohol or drugs, or excessively using alcohol or drugs. This may be over a prolonged or extended period of time, particular in the case of alcohol, e.g. over a period of years. The drugs may be recreational drugs (e.g. cocaine or amphetamines) or anabolic steroids.

In another embodiment the subject may be undergoing cardiotoxic therapy, that is to say medical treatment which is damaging to the heart (i.e. which may cause cardiac remodelling). For instance, many chemotherapy agents commonly used in cancer treatment are cardiotoxic, including anthracyclines (such as doxorubicin), alkylating agents (such as cisplatin, carboplatin and cyclophosphamide) topoisomerase inhibitors, antimetabolites (such as 5-fluorouracil) and monoclonal antibodies. Thus in a particular embodiment the subject is undergoing chemotherapy for treatment of cancer. Radiotherapy, also commonly used in cancer treatment, is also cardiotoxic. Thus in another particular embodiment the subject is undergoing radiotherapy for treatment of cancer. By "undergoing" chemotherapy or radiotherapy is meant a subject who is currently receiving a course of chemotherapy or radiotherapy, who is to begin a course of chemotherapy or radiotherapy (e.g. has been prescribed chemotherapy or radiotherapy), or who has recently completed a course of chemotherapy or radiotherapy. Thus in this context, the polypeptide used according to the present disclosure may be administered to the subject in advance of their chemo/radiotherapy course, during their chemo/radiotherapy course and/or after the completion of their chemo/radiotherapy course.

As described above, the subject treated according to the disclosure may not display any signs of cardiac remodelling at the initiation of treatment. In this embodiment, the subject treated is at risk of cardiac remodelling occurring (e.g. because they are suffering from a condition as described above), and the polypeptidic compound described herein is administered to prevent cardiac remodelling from occurring. Alternatively, the subject treated according to the disclosure may display existing cardiac remodelling, i.e. some cardiac remodelling may have already taken place within the subject prior to the initiation of treatment. In this embodiment, the polypeptidic compound described herein is administered to prevent further cardiac remodelling from occurring. Thus prevention of cardiac remodelling, as defined herein, includes both the prevention of the initiation of cardiac remodelling and also the prevention of further cardiac remodelling, in a subject in whom some remodelling has already taken place. When the subject to be treated is a subject in whom some cardiac remodelling has already taken place, administration of the polypeptidic compound described herein may also treat the cardiac remodelling, that is to say reverse or partially reverse the existing cardiac remodelling.

As detailed above, the subject to be treated according to the disclosure may be at risk of heart failure (but not display any overt symptoms of heart failure). In this embodiment, the subject is treated according to the disclosure to prevent the development of heart failure, or to reduce the risk of heart failure developing. In another embodiment, as detailed above, the subject treated has early stage heart failure (with a severity level of NYHA class I or class II). In this embodiment, the subject is treated according to the disclosure to prevent the early stage heart failure from progressing to moderate or severe heart failure (i.e. with a severity level of NYHA class III or class IV), or to reduce the risk of the heart failure progressing in this manner.

In a particular embodiment, treatment according to the disclosure prevents the development of diastolic dysfunction, or prevents the worsening of diastolic dysfunction. Treatment according to the disclosure may even improve diastolic function. In so doing, the risk of the subject developing heart failure may be reduced. Importantly, as stated above, heart failure with diastolic dysfunction and preserved systolic function is a frequent condition associated with a poor prognosis and for which there is currently a lack of effective treatment. Diastolic function can be assessed by conventional echocardiographic and tissue Doppler examination. A reduced E/A ratio, which may be calculated as described in the Examples, is indicative of reduced diastolic function.

The polypeptidic compound for use according to the disclosure comprises the amino acid sequence set forth in SEQ ID NO: 1, or an amino acid sequence having at least 90 % sequence identity thereto, wherein said compound comprises at least 34 amino acid residues and no more than 50 amino acid residues.

As used herein, the term "polypeptidic compound" means a compound which is composed of amino acids or equivalent subunits, which are linked together by peptide or equivalent bonds. Thus, the term "polypeptidic compound" includes peptides and peptidomimetics.

By "equivalent subunit" is meant a subunit which is structurally and functionally similar to an amino acid. The backbone moiety of the subunit may differ from a standard amino acid, e.g. it may incorporate one or more nitrogen atoms instead of one or more carbon atoms.

By "peptidomimetic" is meant a compound which is functionally equivalent or similar to a peptide and which can adopt a three-dimensional structure similar to its peptide counterparts, but which is not solely composed of amino acids linked by peptide bonds. A preferred class of peptidomimetics are peptoids, i.e. *N*-substituted glycines. Peptoids are closely related to their natural peptide counterparts, but they differ chemically in that their side chains are appended to nitrogen atoms along the molecule's backbone, rather than to the α-carbons as they are in amino acids.

Peptidomimetics typically have a longer half-life within a patient's body, so they are preferred in embodiments where a longer lasting effect is desired. This can help reduce the frequency at which the composition has to be re-administered. However, for bio-safety reasons a shorter half-life may be preferred in other embodiments; in those embodiments peptides are preferred.

Preferably, the polypeptidic compound is a polypeptide. The polypeptidic compound may incorporate D-amino acids, though preferably consists of L-amino acids. The polypeptidic compound may incorporate β-amino acids, though preferably consists of α-amino acids. A peptide consisting wholly of L-amino acids is known in the art as an L-peptide, while a peptide consisting wholly of D-amino acids is known in the art as a D-peptide. The term "inverso-peptide" is used to refer to a peptide with the same amino acid sequence as an L-peptide, but consisting wholly of D-amino acids (i.e. a D-peptide with the same sequence as a corresponding L-peptide). An inverso-peptide has a mirrored structure to its corresponding L-peptide (i.e. an L-peptide of the same amino acid sequence). Inverso-peptides can be advantageous for use in a clinical setting (relative to L-peptides) because they are not generally susceptible to degradation by serum proteases (due to their unnatural conformation inverso-peptides may not be recognised by protease enzymes). A retro-inverso-peptide is a peptide with the reverse sequence of a particular L-peptide, and consisting wholly of D-amino acids. In a particular embodiment the polypeptidic compound for use according to the disclosure is a retro-inverso version of the peptide of SEQ ID NO: 1.

A polypeptide is a polymer formed from amino acids joined to one another by peptide bonds. As defined herein, a polypeptide comprises at least three amino acid residues, though clearly a polypeptidic compound for use according to the disclosure comprises more than three amino acid residues. A polypeptidic compound or polypeptide as defined herein has a maximum length up to 40 or 50 amino acid residues. The polypeptidic compound of the disclosure may thus comprise at least 34 and no more than 50 subunits (e.g. amino acid residues). Alternatively defined it comprises no more than 45, 40 or 37 subunits (e.g. amino acid residues).

A polypeptidic compound as defined herein may be simply a polypeptide, i.e. a polymer consisting of amino acids joined by peptide bonds. Alternatively, the polypeptidic compound may comprise additional functional groups, conjugates, etc. The polypeptidic compound may in particular be linear, e.g. it may be a linear polypeptide. A linear polypeptide is a polypeptide that does not form a ring or lariat structure. Notably, a linear polypeptide may of course contain circular side chains, e.g. in proline residues or aromatic amino acid residues, but in a linear polypeptide the peptide chain itself does not form a ring structure.

The polypeptidic compound for use according to the present disclosure comprises the amino acid sequence set forth in SEQ ID NO: 1, or an amino acid sequence having at least 90 % or 95 % sequence identity thereto. In a particular embodiment, the polypeptidic compound comprises the amino acid sequence set forth in SEQ ID NO: 1. In another embodiment, the polypeptidic compound consists of the amino acid sequence set forth in SEQ ID NO: 1, or an amino acid sequence having at least 90 % or 95 % sequence identity thereto. In another embodiment, the polypeptidic compound consists of the amino acid sequence set forth in SEQ ID NO: 1.

As detailed above, SEQ ID NO: 1 is the human proANP₃₁₋₆₇ (or VSDL) peptide. As described above, the polypeptidic compound for use according to the disclosure may be human proANP₃₁₋₆₇, or it may be a variant of human proANP₃₁₋₆₇ having at least 90 % sequence identity thereto. Variants of proANP₃₁₋₆₇ include derivatives or mimetics of native proANP₃₁₋₆₇, as detailed above. Such variants of proANP₃₁₋₆₇ are suitable for use in the present disclosure providing that the variant of the native peptide does not display any substantial decrease in biological activity relative to the native peptide. In this regard, a "substantial" decrease in biological activity is defined as a decrease in activity of at least 10 % relative to native human proANP₃₁₋₆₇ of SEQ ID NO: 1, as measured by the *in vitro* vasodilation assay (using aortic strips) described by Vesely in US 5,691,310. Variants of proANP₃₁₋₆₇ for use according to the disclosure may include variants of proANP₃₁₋₆₇ comprising conservative amino acid substitutions relative to the native sequence.

The term "conservative amino acid substitution", as used herein, refers to an amino acid substitution in which one amino acid residue is replaced with another amino acid residue having a similar side chain. Amino acids with similar side chains tend to have similar properties, and thus a conservative substitution of an amino acid important for the structure or function of a polypeptide may be expected to affect polypeptide structure/function less than a non-conservative amino acid substitution at the same position. Families of amino acid residues having similar side chains have been defined in the art, including basic side chains (e.g. lysine, arginine, histidine), acidic side chains (e.g. aspartic acid, glutamic acid), uncharged polar side chains (e.g. asparagine, glutamine, serine, threonine, tyrosine), nonpolar side chains (e.g. glycine, cysteine, alanine, valine, leucine, isoleucine, proline, phenylalanine, methionine, tryptophan) and aromatic side chains (e.g. tyrosine, phenylalanine, tryptophan, histidine). Thus a conservative amino acid substitution may be considered to be a substitution in which a particular amino acid residue is substituted for a different amino acid in the same family.

Nonetheless, a variant of proANP₃₁₋₆₇ for use according to the disclosure may comprise a non-conservative amino acid substitution relative to the native peptide, in which one amino acid is substituted for another with a side-chain belonging to a different family, so long as the substitution does not negatively impact on the biological activity of the peptide.

Some specific examples of suitable amino acid substitutions which may be made within proANP₃₁₋₆₇, to yield a variant of proANP₃₁₋₆₇ suitable for use according to the disclosure, may include Pro→Gln (especially at position 41 of proANP; i.e. position 10 of proANP₃₁₋₆₇), Thr→Ala (especially at position 59 of proANP; i.e. position 28 of proANP₃₁₋₆₇), Glu→Asp (especially at position 61 of proANP, i.e. position 30 of proANP₃₁₋₆₇), and Ser→Asn (especially at position 63 of proANP, i.e. position 32 of proANP₃₁₋₆₇).

As noted above, any variant of proANP₃₁₋₆₇ for use according to the disclosure has at least 80 % sequence identity to SEQ ID NO: 1. The level of sequence identity between two sequences (e.g. a polypeptide sequence and the sequence set forth in SEQ ID NO: 1) may be determined by performing a sequence alignment. A sequence alignment may be performed using any suitable method, for instance a computer programme such as EMBOSS Needle or EMBOSS stretcher (both Rice, P. et al., Trends Genet. 16(6): 276-277, 2000) may be used for pairwise sequence alignments while Clustal Omega (Sievers, F. et al., Mol. Syst. Biol. 7:539, 2011) or MUSCLE (Edgar, R.C., Nucleic Acids Res. 32(5):1792-1797, 2004) may be used for multiple sequence alignments. Such computer programmes may be used with the standard input parameters, e.g. the standard Clustal Omega parameters: matrix Gonnet, gap opening penalty 6, gap extension penalty 1; or the standard EMBOSS Needle parameters: matrix BLOSUM62, gap opening penalty 10, gap extension penalty 0.5. Any other suitable parameters may alternatively be used.

A polypeptidic compound as described herein may be synthesised by the skilled person using standard biochemical techniques. If the polypeptidic compound is an L-peptide comprising only proteinogenic amino acids, it may be synthesised by recombinant DNA technology. That is to say, a DNA sequence encoding the polypeptidic compound may be cloned and introduced into an expression vector, which is then introduced into a cellular expression system using standard techniques. Suitable expression systems may include bacterial cells and/or eukaryotic cells such as yeast cells, insect cells or mammalian cells. Given that the polypeptidic compound described herein is derived from a human protein, a eukaryotic cell may be a more appropriate cellular expression system for production of the polypeptidic compound, in particular a mammalian cell. Particularly suitable cells for recombinant expression of the polypeptidic compound may include Chinese hamster ovary (CHO) cells, COS monkey kidney cells and HEK293 cells.

Instead of a cellular expression system, a cell-free, *in vitro* protein expression system may be used to synthesise an L-peptide compound for use according to the disclosure. In such a system a nucleotide sequence encoding the polypeptidic compound is transcribed into mRNA, and the mRNA translated into a protein, *in vitro.* Cell-free expression system kits are widely commercially available, and can be purchased from e.g. Thermo Fisher Scientific (USA).

Polypeptidic compounds for use according to the disclosure may alternatively be chemically synthesised in a non-biological system. Polypeptidic compounds which comprise D-amino acids or other non-proteinogenic amino acids may in particular be chemically synthesised, since biological synthesis is generally not possible in this case. Liquid-phase protein synthesis or solid-phase protein synthesis may be used to generate polypeptides which may form or be comprised within the polypeptidic compounds for use in the disclosure. Such methods are well-known to the skilled person, who can readily produce polypeptidic compounds using appropriate methodology common in the art.

Typically, the polypeptidic compound for use according to the disclosure will be administered as a composition consisting of a solution or suspension of the polypeptidic compound in a pharmaceutically-acceptable carrier, diluent or excipient. However, it will be readily appreciated by the person skilled in the art that the polypeptidic compound may be bound to or associated with a carrier molecule (e.g. a carrier protein or fusion partner such as human serum albumin (HSA), a polysaccharide (e.g. dextran) or a polyether (e.g. polyethylene glycol)) in order to modulate the biological activity and/or serum half-life of the polypeptidic compound.

Suitable pharmaceutically-acceptable diluents, carriers and excipients are well known in the art. For instance, suitable excipients include lactose, maize starch or derivatives thereof, stearic acid or salts thereof, vegetable oils, waxes, fats and polyols. Suitable carriers or diluents include carboxymethylcellulose (CMC), methylcellulose, hydroxypropylmethylcellulose (HPMC), dextrose, trehalose, polyvinyl alcohol, pharmaceutical grade starch, mannitol, lactose, magnesium stearate, sodium saccharin, talcum, cellulose, glucose, sucrose (and other sugars), magnesium carbonate, gelatine, oil, alcohol, detergents and emulsifiers such as polysorbates. Stabilising agents, wetting agents, sweeteners etc. may also be used.

Liquid pharmaceutical compositions, whether they be solutions, suspensions or other like form, may include one or more of the following: sterile diluents such as water, saline solution (preferably physiological, i.e. isotonic), Ringer's solution, fixed oils such as synthetic mono- or diglycerides which may serve as a solvent or suspending medium, polyethylene glycols, glycerine, propylene glycol or other solvents; antibacterial agents such as benzyl alcohol or methyl paraben; antioxidants such as ascorbic acid or sodium bisulfite; chelating agents such as EDTA; buffers such as acetates, citrates or phosphates and agents for the adjustment of tonicity such as sodium chloride or dextrose. A parenteral preparation can be enclosed in ampoules, disposable syringes or multiple dose vials made of glass or plastic. A pharmaceutical composition comprising a polypeptidic compound for use according to the disclosure is preferably sterile.

The administration of the polypeptidic compound according to the disclosure may be performed by any suitable route. For instance, the polypeptidic compound may be administered to the subject intravenously (IV), subcutaneously (sc) or intranasally. IV administration is particularly suitable in the hospital setting, and sc administration and intranasal administration are suitable both within the hospital setting and in the community. Subcutaneous administration of the polypeptidic compound is preferred. The polypeptidic compound is preferably administered as a bolus and/or sustained infusion (e.g. for a period of 30 minutes, 1 hour or longer). For example, infusion may be via a standard catheter or implantable drug port (e.g. a Port-a-Cath^{®}; Smiths Medical MD, Inc., USA), or otherwise achieved using a drug infusion pump (e.g. an implantable drug infusion pump such as an Alzet^{®} osmotic pump (Durect Corporation, USA) or a Duros^{®} device (Intarcia Therapeutics, Inc., USA), or a drug infusion pump for subcutaneous (sc) administration such as a Paradigm^{™} device (Medtronic, USA), all of which can provide a controlled release of the polypeptidic compound. The use of an implantable drug port or drug infusion pump is particularly well suited for long term or extended treatments. Typically, the polypeptidic compound will be infused at a constant rate. However, in some cases it may be desirable to employ a drug infusion pump employing a feedback control mechanism (e.g. a feedback linked to measurement of oedema (in the lung) or other surrogate marker) to control release of the polypeptidic compound.

The dosage of the polypeptidic compound administered according to the disclosure may be determined by factors such as the medical condition of the patient (e.g. what medical conditions the patient suffers from). Appropriate dosages may be determined as a factor of the size of the patient. The skilled clinician will be able to calculate an appropriate dose for a patient based on all relevant factors, e.g. age, height, weight, the condition to be treated and its severity.

As shown in the Examples below, the present inventors have discovered that the polypeptide of SEQ ID NO: 1 has a cardioprotective effect (i.e. prevents cardiac remodelling) independent of its antihypertensive effect. Even at low dosages where no antihypertensive effect is seen, the polypeptide of SEQ ID NO: 1 has a cardioprotective effect in a model of hypertension. In a preferred embodiment of the disclosure the polypeptidic compound is administered at a dosage such that it has a cardioprotective effect (prevents cardiac remodelling) without affecting the subject's blood pressure. Such an effect is seen with the polypeptide of SEQ ID NO: 1 when it is at a plasma concentration in the range 0.15 to 10 ng/ml. In an embodiment, the polypeptidic compound is administered to the subject at a dosage suitable or appropriate or selected to achieve a plasma concentration of the compound in the subject in the range 0.15 to 10 ng/ml, most preferably at a dosage to achieve plasma concentration of the compound in the subject in the range 0.15 to 2 ng/ml. A plasma concentration of the polypeptidic compound of the disclosure (e.g. the polypeptide of SEQ ID NO: 1) in the range 0.15 to 10 ng/ml may be achieved by subcutaneous administration of 25 to 2000 ng/kg/day of the polypeptidic compound (by ng/kg/day is meant ng per kg body mass of the subject to be treated per day).

Accordingly, in an embodiment of the disclosure the polypeptidic compound is administered subcutaneously to the subject at a dosage in the range 25 to 2000 ng/kg/day. In other embodiments the maximum subcutaneous dosage may be 1900, 1800, 1700, 1600, 1500, 1400, 1300, 1200, 1100, 1000, 900, 800, 700, 600, 500, 400, 300, 200, 100, 95, 90, 85, 80, 75, 70, 65, 60, 55 or 50 ng/kg/day. In other embodiments the minimum subcutaneous dosage may be 30, 35, 40, 45, 50, 55, 60, 65, 70, 75, 80, 85, 90, 95, 100, 200, 300, 400, 500, 600, 700, 800, 900, 1000, 1100, 1200, 1300, 1400 or 1500 ng/kg/day. In some embodiments of the disclosure, the polypeptidic compound is administered subcutaneously to the subject at a dosage in the range 25 to 50 ng/kg/day, 25 to 75 ng/kg/day, 25 to 80 ng/kg/day, 25 to 85 ng/kg/day, 25 to 90 ng/kg/day, 25 to 95 ng/kg/day, 25 to 100 ng/kg/day, 25 to 200 ng/kg/day, 25 to 500 ng/kg/day, 25 to 1000 ng/kg/day or 25 to 1500 ng/kg/day.

In other embodiments of the disclosure, the polypeptidic compound is administered to the subject subcutaneously at a dosage in the range 50 to 75 ng/kg/day, 50 to 80 ng/kg/day, 50 to 85 ng/kg/day, 50 to 90 ng/kg/day, 50 to 95 ng/kg/day, 50 to 100 ng/kg/day, 50 to 200 ng/kg/day, 50 to 500 ng/kg/day, 50 to 1000 ng/kg/day or 50 to 1500 ng/kg/day. In other embodiments of the disclosure, the polypeptidic compound is administered to the subject subcutaneously at a dosage in the range 100 to 200 ng/kg/day, 100 to 300 ng/kg/day, 100 to 400 ng/kg/day, 100 to 500 ng/kg/day, 100 to 1000 ng/kg/day, 100 to 1500 ng/kg/day or 100 to 2000 ng/kg/day. In other embodiments of the disclosure, the polypeptidic compound is administered to the subject subcutaneously at a dosage in the range 250 to 500 ng/kg/day, 250 to 750 ng/kg/day, 250 to 1000 ng/kg/day, 250 to 1500 ng/kg/day or 250 to 2000 ng/kg/day. In other embodiments of the disclosure, the polypeptidic compound is administered to the subject subcutaneously at a dosage in the range 500 to 1000 ng/kg/day, 500 to 1500 ng/kg/day or 500 to 2000 ng/kg/day. In other embodiments of the disclosure, the polypeptidic compound is administered to the subject subcutaneously at a dosage in the range 1000 to 1500 ng/kg/day or 1000 to 2000 ng/kg/day.

In preferred embodiments of the disclosure, the polypeptidic compound is administered to the subject subcutaneously at a dosage less than 100 ng/kg/day, e.g. at a dosage in the range 25 to 50 ng/kg/day, 25 to 75 ng/kg/day, 25 to 80 ng/kg/day, 25 to 85 ng/kg/day, 25 to 90 ng/kg/day or 25 to 95 ng/kg/day.

The target plasma concentration of the polypeptidic compound may alternatively be achieved by intravenous administration of 5 to 1000 ng/kg/day of the polypeptidic compound. Accordingly, in an embodiment of the disclosure the polypeptidic compound is administered intravenously to the subject at a dosage in the range 5 to 1000 ng/kg/day. In other embodiments the maximum intravenous dosage may be 900, 800, 700, 600, 500, 400, 300, 200, 100, 95, 90, 85, 80, 75, 70, 65, 60, 55, 50, 45, 40, 35, 30, 25 or 20 ng/kg/day. In other embodiments the minimum intravenous dosage may be 10, 15, 20, 25, 30, 35, 40, 45, 50, 55, 60, 65, 70, 75, 80, 85, 90, 95, 100, 200, 300, 400 or 500 ng/kg/day.

In some embodiments of the disclosure, the polypeptidic compound is administered to the subject intravenously at a dosage in the range 5 to 15 ng/kg/day, 6 to 18 ng/kg/day, 6 to 20 ng/kg/day, 6 to 23 ng/kg/day, 6 to 25 ng/kg/day, 6 to 30 ng/kg/day, 6 to 40 ng/kg/day, 6 to 50 ng/kg/day, 6 to 75 ng/kg/day, 6 to 100 ng/kg/day, 6 to 250 ng/kg/day or 6 to 500 ng/kg/day. In other embodiments the polypeptidic compound is administered to the subject intravenously at a dosage in the range 10 to 18 ng/kg/day, 10 to 20 ng/kg/day, 10 to 25 ng/kg/day, 10 to 30 ng/kg/day, 10 to 40 ng/kg/day, 10 to 50 ng/kg/day, 10 to 75 ng/kg/day, 10 to 100 ng/kg/day, 10 to 250 ng/kg/day or 10 to 500 ng/kg/day. In other embodiments the polypeptidic compound is administered to the subject intravenously at a dosage in the range 25 to 50 ng/kg/day, 25 to 75 ng/kg/day, 25 to 100 ng/kg/day, 25 to 250 ng/kg/day or 25 to 500 ng/kg/day.

In preferred embodiments of the disclosure, the polypeptidic compound is administered to the subject intravenously at a dosage up to 25 ng/kg/day, e.g. at a dosage in the range 5 to 10 ng/kg/day, 5 to 15 ng/kg/day, 5 to 20 ng/kg/day, 5 to 25 ng/kg/day, 6 to 10 ng/kg/day, 6 to 15 ng/kg/day, 6 to 18 ng/kg/day, 6 to 20 ng/kg/day, 6 to 23 ng/kg/day, 6 to 25 ng/kg/day, 10 to 15 ng/kg/day, 10 to 20 ng/kg/day or 10 to 25 ng/kg/day.

The target plasma concentration of the polypeptidic compound may alternatively be achieved by intranasal administration of 5 to 1000 ng/kg/day of the polypeptidic compound. Accordingly, in an embodiment of the disclosure the polypeptidic compound is administered intranasally to the subject at a dosage in the range 5 to 1000 ng/kg/day. Accordingly, in an embodiment of the disclosure the polypeptidic compound is administered intranasally to the subject at a dosage in the range 5 to 1000 ng/kg/day. In other embodiments the maximum intranasal dosage may be 900, 800, 700, 600, 500, 400, 300, 200, 100, 95, 90, 85, 80, 75, 70, 65, 60, 55, 50, 45, 40, 35, 30, 25 or 20 ng/kg/day. In other embodiments the minimum intranasal dosage may be 10, 15, 20, 25, 30, 35, 40, 45, 50, 55, 60, 65, 70, 75, 80, 85, 90, 95, 100, 200, 300, 400 or 500 ng/kg/day.

In some embodiments of the disclosure, the polypeptidic compound is administered to the subject intranasally at a dosage in the range 5 to 15 ng/kg/day, 5 to 20 ng/kg/day, 5 to 25 ng/kg/day, 5 to 30 ng/kg/day, 5 to 40 ng/kg/day, 5 to 50 ng/kg/day, 5 to 75 ng/kg/day, 5 to 100 ng/kg/day, 5 to 250 ng/kg/day or 5 to 500 ng/kg/day. In other embodiments the polypeptidic compound is administered to the subject intranasally at a dosage in the range 8 to 15 ng/kg/day, 8 to 20 ng/kg/day, 8 to 25 ng/kg/day, 8 to 30 ng/kg/day, 8 to 40 ng/kg/day, 8 to 50 ng/kg/day, 8 to 75 ng/kg/day, 8 to 100 ng/kg/day, 8 to 250 ng/kg/day or 8 to 500 ng/kg/day. In other embodiments the polypeptidic compound is administered to the subject intranasally at a dosage in the range 10 to 20 ng/kg/day, 10 to 25 ng/kg/day, 10 to 30 ng/kg/day, 10 to 40 ng/kg/day, 10 to 50 ng/kg/day, 10 to 75 ng/kg/day, 10 to 100 ng/kg/day, 10 to 250 ng/kg/day or 10 to 500 ng/kg/day. In other embodiments the polypeptidic compound is administered to the subject intranasally at a dosage in the range 25 to 50 ng/kg/day, 25 to 75 ng/kg/day, 25 to 100 ng/kg/day, 25 to 250 ng/kg/day or 25 to 500 ng/kg/day.

In preferred embodiments of the disclosure, the polypeptidic compound is administered to the subject intranasally at a dosage up to 30 ng/kg/day, e.g. at a dosage in the range 5 to 10 ng/kg/day, 5 to 15 ng/kg/day, 5 to 20 ng/kg/day, 5 to 25 ng/kg/day, 5 to 30 ng/kg/day, 8 to 15 ng/kg/day, 8 to 20 ng/kg/day, 8 to 25 ng/kg/day, 8 to 30 ng/kg/day, 10 to 15 ng/kg/day, 10 to 20 ng/kg/day, 10 to 25 ng/kg/day or 10 to 30 ng/kg/day.

In particular, based on the known pharmacokinetics of proANP₃₁₋₆₇ it has been calculated that subcutaneous administration of 25 to 95 ng/kg/day, intravenous administration of 6 to 23 ng/kg/day and intranasal administration of 8 to 30 ng/kg/day of the peptide can be expected to yield approximately the same plasma concentrations of the peptide. In a preferred embodiment of the disclosure, proANP₃₁₋₆₇ is administered to the subject subcutaneously at a dosage in the range 25 to 95 ng/kg/day, intravenously at a dosage in the range 6 to 23 ng/kg/day or intranasally in the range 8 to 30 ng/kg/day. Similarly, it has been calculated that subcutaneous administration of 25 to 75 ng/kg/day, intravenous administration of 6 to 18 ng/kg/day and intranasal administration of 8 to 25 ng/kg/day of the proANP₃₁₋₆₇ peptide can be expected to yield approximately the same plasma concentrations of the peptide. In another preferred embodiment of the disclosure, proANP₃₁₋₆₇ is administered to the subject subcutaneously at a dosage in the range 25 to 75 ng/kg/day, intravenously at a dosage in the range 6 to 18 ng/kg/day or intranasally in the range 8 to 25 ng/kg/day.

The polypeptidic compound for use according to the disclosure may be administered to the subject in combination with one or more other therapeutic agents. In particular, if the subject to be treated has a medical condition which puts them at risk of heart failure, as described above, the subject may be administered the polypeptidic compound according to the disclosure in combination with one or more therapeutic agents used for treatment of their existing medical condition. The therapeutic agent used for treatment of the existing medical condition acts to treat the existing medical condition, e.g. alleviate its symptoms, etc., while the polypeptidic compound for use according to the disclosure acts to prevent or treat cardiac remodelling which has been or may be caused by the medical condition in question. Thus for example, as described above, a hypertensive subject may be administered the polypeptidic compound according to the disclosure in combination with one or more antihypertensive agents.

In a particular embodiment the polypeptidic compound used according to the disclosure is not administered to a subject in combination with a diuretic agent. A diuretic agent is a drug which increases the production of urine. Diuretic agents include loop diuretics, thiazides, carbonic anhydrase inhibitors, potassium-sparing diuretics, calcium-sparing diuretics, osmotic diuretics and low ceiling diuretics. Some diuretic agents may fall into multiple categories. Diuretic agents are used in medicine for the treatment of a number of conditions, including heart failure and chronic kidney disease. In a particular embodiment, the subject to be treated is not administered a diuretic agent at the same time, or within the same dosing schedule, as he or she is administered the polypeptidic compound.

The disclosure may be further understood by reference to the non-limiting examples below, and the figures.

### Figure Legends:

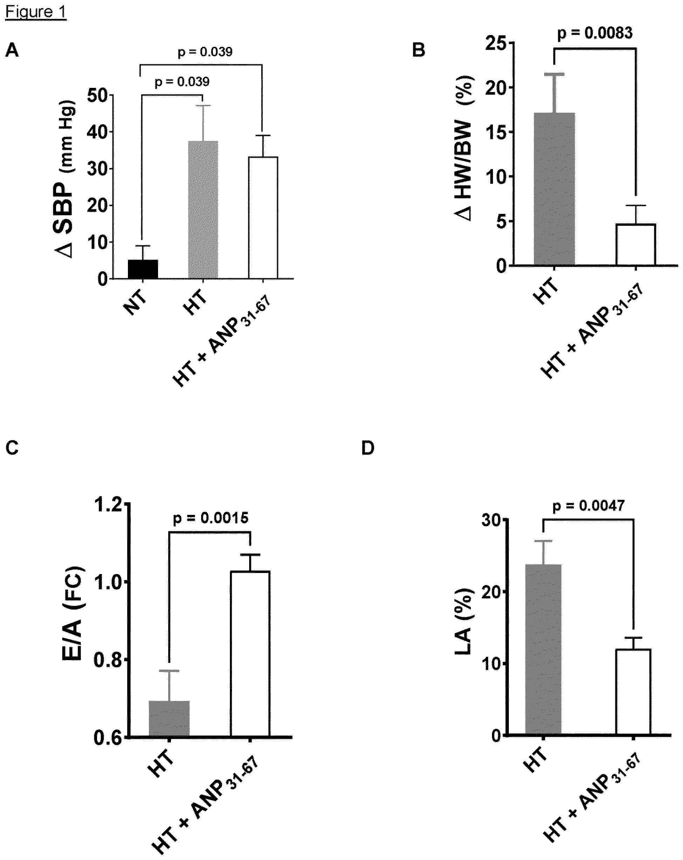

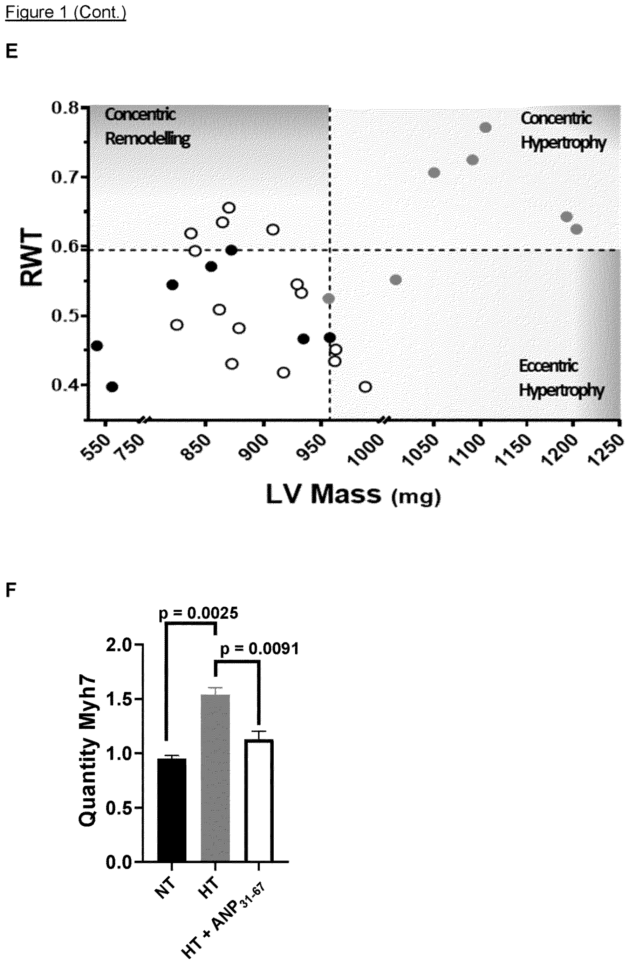

*Figure 1**: Effects of proANP₃₁₋₆₇ on Heart Structure and Function*
   **(A)** Δ SBP indicates the difference in systolic blood pressure at 6 weeks relative to baseline, within each group. Δ SBP was higher in both groups fed on a high-salt diet (the hypertensive (HT) groups) compared to the group fed on a normal salt diet (the normotensive (NT) group). No difference in Δ SBP was observed between untreated HT rats and those treated with proANP₃₁₋₆₇.
   **(B)** Cardiac hypertrophy in HT rats is indicated by the percentage increase in HW/BW (relative to NT rats). Untreated HT rats exhibited an increase in HW/BW of more than 15 %, whereas proANP₃₁₋₆₇-treated rats exhibited almost no increase in HW/BW.
   **(C)** The E/A ratio is the ratio of peak velocity blood flow from gravity in early diastole (the E wave) to peak velocity flow in late diastole caused by atrial contraction (the A wave); The decrease in the E/A ratio in untreated HT rats (relative to NT controls) indicated that the hearts of the untreated HT rats had become stiffer, whereas proANP₃₁₋₆₇-treated HT rats had an essentially unchanged E/A ratio, indicating preserved diastolic function.
   **(D)** LA (%) indicates the percentage left atrium (LA) enlargement (measured by the increase in LA diameter) relative to NT rats, secondary to hypertension. Much greater LA enlargement was seen in the HT untreated group than in the proANP₃₁₋₆₇-treated group.
   **(E)** Approximately 70 % of the untreated HT rats (grey circles) developed concentric hypertrophy (top-right quadrant), whereas about 70 % of the proANP₃₁₋₆₇-treated HT rats (white circles) had normal geometry (bottom-left quadrant). All NT rats (black circles) had normal cardiac geometry at 6 weeks (bottom-left quadrant). RWT is relative wall thickness, and LV mass is left ventricle mass.
   **(F)** The level of Myh7 expression was measured by qPCR. As shown, Myh7 expression is essentially unchanged in treated HT rats relative to NT rats, but significantly higher in untreated HT rats than in NT rats and treated HT rats.

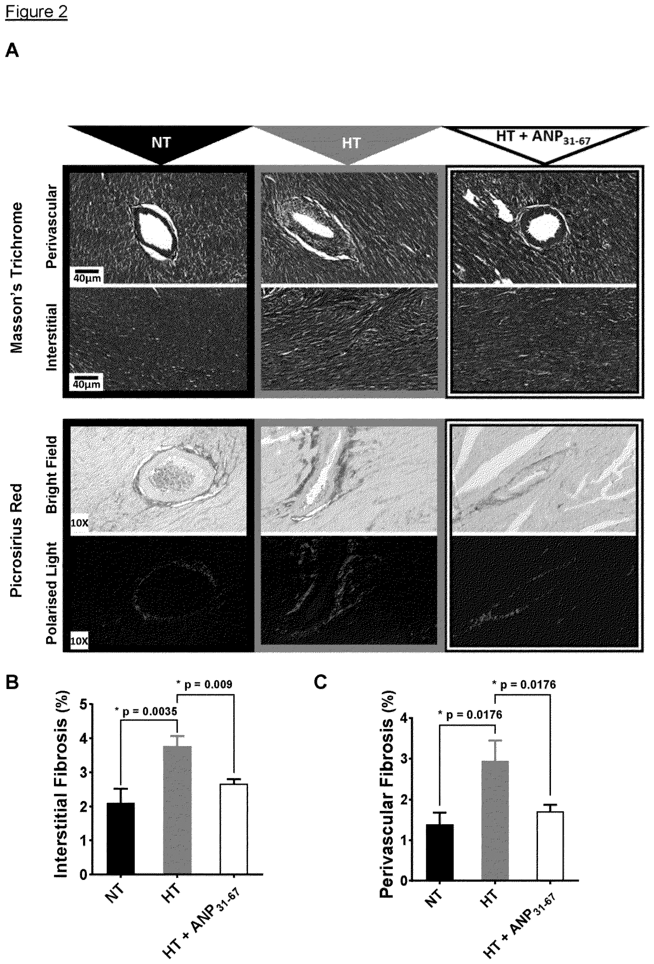

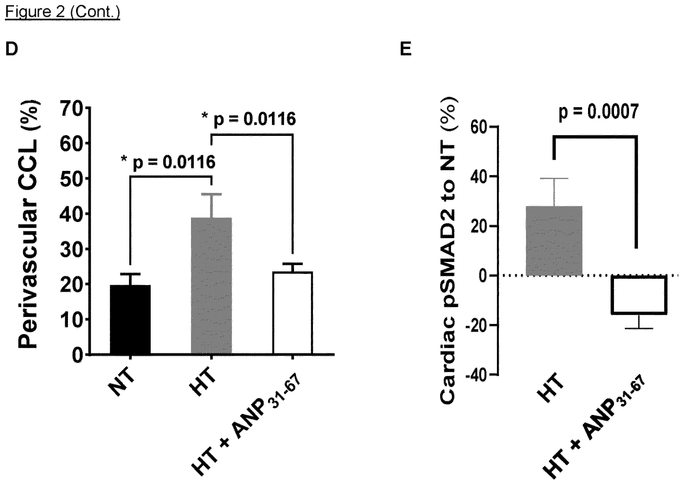

*Figure 2**: Effects of proANP₃₁₋₆₇ on Cardiac Collagen Deposition and Maturation*
   **(A)** Illustrative images of the interstitial and perivascular left ventricle (LV) areas obtained by Masson's trichrome staining (top), and of LV perivascular collagen cross-linking (CCL) obtained by Picrosirius Red staining (bottom). Scale bars in top panel indicate distance of 40 µm. Images in bottom panel are 10x magnified.
   **(B, C)** Cardiac collagen deposition was elevated in both interstitial and perivascular LV areas of the untreated HT rats compared to NT rats. ProANP₃₁₋₆₇-treated rats had significantly reduced fibrosis compared to HT untreated rats.
   **(D)** Untreated HT rats had elevated levels of LV perivascular CCL compared to NT rats. ProANP₃₁₋₆₇-treated rats had significantly lower perivascular CCL compared to untreated HT rats (essentially unchanged relative to NT rats).
   **(E)** Levels of nuclear phosphorylated SMAD2 were measured by quantitative Western blot. Levels of nuclear pSMAD2 in HT and treated HT rats are shown relative to levels in NT rats. Nuclear pSMAD2 levels were substantially higher in HT rats than NT rats, but slightly lower in treated HT rats than NT rats.

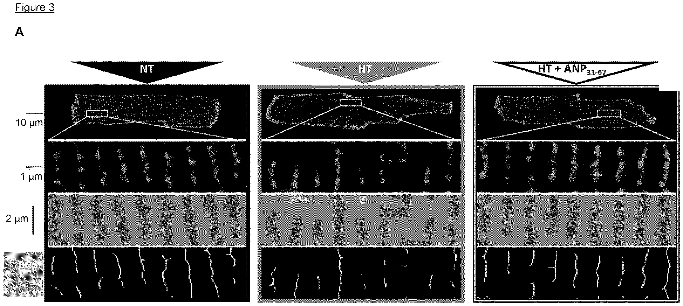

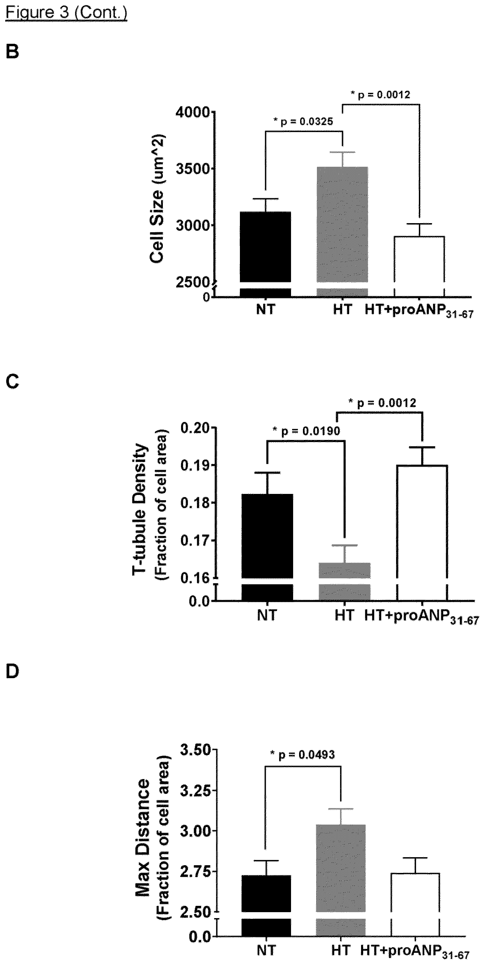

*Figure 3**: ProANP₃₁₋₆₇ Preserves LV Cardiomyocyte Size, t-Tubule Structure and Density*
   **(A)** t-tubules were visualized with di-8-ANEPPS in the three Dahl/SS rat groups (upper panels). Distances from all points in the cytosol to the nearest t-tubule or surface sarcolemma were also obtained together with the fraction of transversely-oriented tubules (Trans.) and longitudinally-oriented tubules (Longi.) (bottom panels). Scale bars are shown for the upper 3 rows of panels.
   **(B)** Cardiomyocyte size was increased in untreated HT rats compared to NT rats, whereas it was unchanged in proANP₃₁₋₆₇-treated HT rats.
   **(C)** t-tubule density was lower in hypertrophic hearts of untreated HT rats than in NT rats, but was preserved in proANP₃₁₋₆₇-treated rats.
   **(D)** Consistent with the reduction in t-tubule density, untreated HT rats had a longer distance among t-tubules compared to NT rats, whereas proANP₃₁₋₆₇-treated rats maintained maximum intracellular distances between t-tubules and the sarcolemmal membrane. (n of NT cells = 60 from 4 hearts, n of untreated HT cells = 60 from 4 hearts, n of proANP₃₁₋₆₇-treated HT cells = 53 from 3 hearts.)
*Figure 4**: ProANP₃₁₋₆₇ Blocks ANGII-Induced Cardiac Hypertrophy*
   Rat neonatal cardiomyocytes were stimulated for 24h with ANGII to induce hypertrophy *in vitro.* ProANP₃₁₋₆₇ at 37.5 ng/ml and 150 ng/ml blocked the pathological actions of ANGII.
*Figure 5**: Effects of Low Dosage proANP₃₁₋₆₇ on Heart Structure and Function*
   **(A)** Δ SBP indicates the difference in systolic blood pressure at 6 weeks relative to baseline, within each group. Δ SBP was higher in both groups fed on a high-salt diet (the hypertensive (HT) groups) compared to the group fed on a normal salt diet (the normotensive (NT) group). No difference in Δ SBP was observed between untreated HT rats and those treated with proANP₃₁₋₆₇.
   **(B)** HW/BW is presented for three groups of rats (NT, untreated HT and HT treated with low dosage (25 ng/kg/day) proANP₃₁₋₆₇). An increase in HW/BW is indicative of cardiac hypertrophy. As shown in Fig. 1, untreated HT rats exhibited an increase in HW/BW of more than 15 % relative to NT rats, whereas HT rats treated with a low dosage of proANP₃₁₋₆₇ exhibited no increase in HW/BW.
   **(C)** As shown in Fig. 1, the E/A ratio in untreated HT rats (relative to NT controls) is reduced, indicating that the hearts of the untreated HT rats had become stiffer. HT rats treated with a low dosage of proANP₃₁₋₆₇ had an essentially unchanged E/A ratio, indicating preserved diastolic function.
   **(D)** The LA diameter was greater in the untreated HT group compared to the NT group, secondary to hypertension. An increase in LA diameter was also seen in the HT group treated with a low dose of proANP₃₁₋₆₇, but this increase was significantly less than in the untreated group.
   **(E)** As shown in Fig. 1, approximately 70 % of the untreated HT rats (grey circles) developed concentric hypertrophy (top-right quadrant), and all NT rats (black circles) had normal cardiac geometry at 6 weeks (bottom-left quadrant). All HT rats treated with low dosage proANP₃₁₋₆₇ (white circles) also had normal geometry (bottom-left quadrant).
   **(F)** The level of Myh7 expression was measured by qPCR. As shown, Myh7 expression is essentially unchanged in treated HT rats relative to NT rats, but significantly higher in untreated HT rats than in NT rats and treated HT rats.

### Examples:

### Example 1: Effect of proANP₃₁₋₆₇ at 50 or 100 ng/kg/day on Hypertensive Rats

### Methods

### Materials

ProANP₃₁₋₆₇ was obtained from Madeline Pharmaceuticals Pty Ltd. (Mount Barker, SA, Australia). The drug manufacturer recommends using 200 mM NaCl with 10 mM sodium acetate buffer (pH 5.5) as vehicle for the drug. Teklad high salt (4.0 % NaCl) diet was obtained from Envigo (TD.92034; Madison, Wl, USA). Normal salt (0.3 % NaCl) diet was from SDS (Special Diets Services; UK).

### Experimental Groups

A total of 29 adult Dahl salt-sensitive (Dahl/SS) male rats with an initial weight of 150 to 200 g were included in the study. Rats were purchased from Charles River Laboratories (USA) and housed in a room with a 12/12-hour light cycle, a temperature of 21°C, and a humidity of 55 %. Rats were maintained on a normal salt diet up to seven weeks of age. Twenty-two Dahl/SS were then randomly switched to a high salt diet for 6 weeks to induce hypertension, whilst 7 rats were kept normotensive (NT) on normal salt diet. Drinking water and food was provided *ad libitum.*

After 2 weeks of high salt diet, 15 hypertensive (HT) rats were started on treatment with proANP₃₁₋₆₇. Rats treated with proANP₃₁₋₆₇ received one of two dosages: 50 ng/kg/day (n = 8) or 100 ng/kg/day (n = 7). A total of 14 rats received only vehicle: control rats on a normal salt diet (n = 7); and HT rats on a high salt diet (n = 7). Rats were randomly assigned to the 3 groups (i.e. control normal salt; high salt untreated; high salt plus treatment). Post-analysis of the treated groups revealed that there was no dose-dependent effects between treatment with 50 or 100 ng/kg/day proANP₃₁₋₆₇, hence treated rats in this example were pooled and analysed as a single group, named "HT + proANP₃₁₋₆₇" ("hypertensive treated").

Drug or vehicle was delivered via Alzet osmotic mini-pumps (Model 2004; mean pumping rate 2.28 ± 0.07 µL/hr, mean fill volume 1997.6 ± 18.3 µL) implanted subcutaneously (as instructed by the manufacturer) for 28 days. Animals were treated with buprenorphine (0.05 mg/kg s.c.) as analgesic 30 minutes before and up to 1 day following implantation of pumps.

### Study Protocol

The study conformed to the regulations governing the laboratory animal facility (Comparative Medicine-Ullevål, OUS, Norway). The protocol was approved by The Norwegian Food Safety Authority committee (Mattilsynet) for animal research (FOTS protocol number 12582).

### Echocardiography

Cardiac function was assessed by transthoracic echocardiography using a VEVO 2100 high resolution *in vivo* imaging system from VisualSonics (Canada). Briefly, animals were maintained under anesthesia (1.5-2 % isoflurane mixed with oxygen) on a pre-warmed ECG transducer pad with body temperature and ECG monitored. Measurements were made with an MS250 transducer, frequency set at 24 MHz. M-mode in the parasternal long axis view was performed to assess the function and dimension of the left ventricle and left atrium. LVEF was calculated as 100 × ((LV Vol;d - LV Vol;s) / LV Vol;d). LV mass was estimated by the formula: 1.053 × ((LVID;d + LVPW;d + IVS;d)3 - LVID;d3). Relative wall thickness (RWT) was calculated as 2 × LVPW;d / LVID;d.21 Normal geometry of the heart was defined as the 95^{th} percentile for both LV Mass and RWT of the NT group. E and A waves in left ventricular filling velocities were assessed via pulsed-wave Doppler in a parasternal long axis view. Echocardiographic analysis were performed by an operator blinded to treatment group.

### Blood Pressure Measurement

Measurement was carried out using the CODA non-invasive blood pressure acquisition system for rats (Kent Scientific Corporation). Animals were kept in restraint tubes and placed over a heating platform (preheated to 33 to 35°C) and blood pressure measured by a tail-cuff system. Each recording session consisted of 25 acclimation cycles (not used in the analysis), followed by 20 inflation and deflation cycles (the occlusion cuff is inflated to 250 mm Hg and deflated over 20 s). Rats were trained for at least 5 consecutive days before blood pressure measurements were recorded.

### Histochemistry

Hearts were excised, rinsed in PBS, quickly blotted on gauze, and then fixed in 10 % formalin for a minimum of 24 hr. The bi-ventricular apex of the heart was embedded in paraffin and cut into 4 µm sections. Sections were stained with Masson's trichrome (Polysciences, Inc., Warrington, PA, USA) to assess collagen abundance. Stained sections were scanned (20x magnification) with AxioScan Z1 (Carl Zeiss), to obtain whole cross-sections for collagen quantification. Total fibrosis area (%) and perivascular fibrosis (ratio of the area of fibrosis surrounding the vessel wall to the lumen area) were quantified using ZEN2 blue edition (Zeiss, Jena, Germany). In addition, heart sections were stained with Picrosirius Red (Polysciences, Inc.) and visualized under bright-field and polarized light (10x magnification) to assess cross-linked collagen. The degree of cross-linked collagen was calculated as the ratio between cross-linked collagen and total collagen. All histological quantifications were independently performed by three trained researchers blinded to rat groups.

### Gene expression

Total RNA was extracted from left ventricle tissue using an RNeasy Fibrous Tissue Mini Kit (Qiagen, Cat. #74704). RNA concentration and quality was assessed by NanoDrop ND-1000 Spectrophotometer (Thermo Fisher Scientific). cDNA synthesis was performed using an iSCRIPT synthesis kit (Bio-Rad). Transcript levels of Mhy7 (Rn00568328_m1) and Rpl32 (Rn00820748_g1) were determined using TaqMan assays (Applied Biosystems) detected on a QuantStudio3 (Thermo Fisher Scientific) and analysed using QuantStudioTM Design (Thermo Fisher Scientific). Mhy7 mRNA levels were calculated by the ΔΔCt method and normalized to Rpl32 transcript value.

### Western blot

Enriched nuclear protein fraction from left ventricle tissue was isolated using Compartment Protein Extraction Kit (Merck Millipore, Cat. #2145), according to the manufacturer's specifications. Protein concentration was measured by BCA assay kit (Thermo Fisher Scientific, Cat. #23225). The tissue lysates were subjected to (4-15% precast polyacrylamide) SDS-PAGE electrophoresis, and the proteins were transferred to PVDF membranes using the iBlot^{®} system (Thermo Fisher Scientific). Membranes were analysed with rabbit anti-SMAD2/3 (1:1000, Cell Signaling Technology, Cat. #8685) and rabbit anti-phospho-SMAD2 (Ser465/467) (1:500, Cell Signaling Technology, Cat. #3108). The membranes were developed using the ECL system (Pierce Protein Research Products) and ChemiDoc XRS (Bio-Rad), and quantified using Image LabTM software (version 6.0.1, Bio-Rad).

### Cardiomyocyte Isolation and t-Tubule Structure

A subset of rats was used for analyses of t-tubule structure in isolated left ventricular cardiomyocytes. These animals were anaesthetised as described above and euthanised by removal of the heart. The excised heart was then placed in cool buffer containing 130 mM NaCl, 25 mM HEPES, 5.4 mM KCI, 0.5 mM MgCl2, 0.4 mM NaH₂PO₄, and 5.5 mM D-glucose (pH 7.4), before mounting on a Langendorff setup for retrograde perfusion though the aorta.

Cardiomyocytes were then isolated as previously described (Frisk et al., American Journal of Physiology - Heart and Circulatory 307: H609-620, 2014). In brief, hearts were perfused with 200 U/mL collagenase type II (Worthington Biochemical, Lakewood, NJ) at 37°C for 15 min. Thereafter, the LV was cut out, minced, and triturated with a cut-off pipette before filtering the solution through a 200 µm nylon-mesh. t-tubules were stained with 10 µM di-8-ANEPPS for 20 min prior to imaging using an LSM800 Airyscan confocal microscope (Zeiss) with a 63× oil-immersed objective. Fluorescence was excited at 488 nm and emitted light above 500 nm was measured. t-tubule captures were recorded with 1871×1871-pixel XY images, recorded as z-stacks (spatial resolution: 77×77×500nm). Image sequences were deconvolved with Huygens Essential software before analysis. Cell area, t-tubule density and distance to nearest t-tubule or sarcolemmal membrane were analysed as previously described (Frisk et al., Cardiovascular Research 112: 445-451, 2016).

### Primary Cultures of Neonatal Cardiac Myocytes

Primary cultures of rat cardiomyocyte cells were prepared as previously described (Larsen et al., Cardiovascular Research 80(1): 47-54, 2008). In brief, hearts from 1-3 day old Wistar rats (Taconic), were isolated by collagen and pancreatin digestion. Cardiomyocytes were separated from noncardiomyocytes by differential attachment to uncoated culture flasks (90151; Techno Plastic Products). Cardiomyocytes were allowed to attach to 6- or 12-well culture plates (Corning International, Corning, NY) coated with gelatine/fibronectin (G-1890/ F1141; Sigma, St, Louis, MO) overnight in plating medium (2 or 1 ml) at a density of 2.5 × 10⁵ cells/ml medium [DMEM (41965; GIBCO-BRL, Invitrogen) supplemented with penicillin/streptomycin/glutamine (G6784; Sigma), medium 199 (31150; GIBCO-BRL), HEPES (15630; GIBCO-BRL), horse serum (14-703E; Bio-Whittaker, Lonza), and fetal calf serum (14-701E; Bio-Whittaker)]. The cardiomyocytes were maintained in plating medium without serum before being stimulated with 1 µM ANG II (A9525; Sigma), proANP₃₁₋₆₇ 37.5 ng/mL or 150 ng/mL, or vehicle for 24 h, and washed twice with DPBS (BE17-512F; BioWhittaker) before being harvested for analysis.

### In Vitro Leucine Incorporation in Neonatal Cardiomyocytes

5 µCi/ml [3H]leucine (American Radiolabeled Chemicals) were added at the same time as ANG II and the cells were washed six times in 95 % EtOH before being harvested in 0.2 M NaOH 24 h after stimulation, as previously described (Halvorsen et al., Journal of Lipid Research 39(4): 901-912, 1998). Serum-stimulated cells served as positive control. [3H]leucine incorporation was quantified by measuring counts per minute in duplicates from each sample with the Wallac Winspectral 1414 liquid scintillation counter (PerkinElmer). Samples were diluted in Pico-Fluor 40 (cat. no. 6013349; PerkinElmer).

### Statistics

Comparisons between 2 groups were made with an unpaired 2-tailed t-test. Comparisons between >2 groups were made with a 1-way ANOVA followed by Holm-Sidak post-hoc test. P values for each comparison are shown in each respective figure, and P<0.05 was considered statistically significant. Values are reported as mean ± SEM. All statistical tests were performed with GraphPad Prism 8.0.1 (San Diego, CA).

### Results

### Blood Pressure

Dahl/SS rats fed a high salt diet showed elevated systolic blood pressure, which was not lowered by proANP₃₁₋₆₇ (Table 1; Fig. 1A). Diastolic blood pressure tended (p = 0.068) to increase only in the proANP₃₁₋₆₇-treated HT rats. Mean arterial blood pressure was also increased only in proANP₃₁₋₆₇-treated HT rats compared to NT. No difference in blood pressure levels between the two HT groups was seen.

### Cardiac Structure and Function

Dahl/SS rats exhibited characteristic signs of adverse cardiac remodeling and function after 6 weeks of high salt diet (Table 1; Fig. 1B-D & 2A-D). Autopsy demonstrated increased cardiac hypertrophy in the untreated HT animals, as indicated by an increase in heart weight to body weight ratio. Heart weight to body weight ratio in proANP₃₁₋₆₇-treated rats was similar to NT (Table 1). The percentage increase in heart weight/body weight compared to NT was more than 15 % for untreated HT rats, and less than 5 % for proANP₃₁₋₆₇-treated rats (Fig. 1B). ProANP₃₁₋₆₇ also attenuated the expression of the Myh7 (MHC-β) gene associated with adverse cardiac hypertrophy (Fig. 1F).

Echocardiographic examination showed preserved systolic function in all groups, as assessed by left ventricular ejection fraction and fractional shortening (Table 1). Cardiac stiffness increased in untreated HT rats, as indicated by a decrease in E/A ratio, but was preserved in proANP₃₁₋₆₇-treated rats (Table 1). Relative fold change to NT controls indicated that the E/A ratio dropped by approximately 50 % in untreated HT rats, whereas the E/A ratio was preserved in proANP₃₁₋₆₇-treated rats (Fig. 1C). Enlarged left atria were observed in both HT groups (Table 1). The left atrium diameter was more than 20 % larger in untreated HT rats compared to NT rats, but only about 10 % larger in proANP₃₁₋₆₇-treated rats compared to NT rats (Fig. 1D).

Cardiac ultrasound revealed that untreated HT animals had increased cardiac wall thickening, which was attenuated in proANP₃₁₋₆₇-treated rats. Both the LV mass and RWT in proANP₃₁₋₆₇-treated rats were similar to in NT rats (Table 1; Fig. 1E). More than 70 % of the untreated HT rats exhibited concentric hypertrophy (Fig. 1E, top-right quadrant), while approximately 70 % of the proANP₃₁₋₆₇-treated HT rats showed normal geometry (Fig. 1E, bottom-left quadrant).

Interstitial and perivascular LV fibrosis increased by 79 % and 78 %, respectively, in the untreated HT rats compared to NT rats. ProANP₃₁₋₆₇ treatment alleviated the development of both forms of fibrosis with a modest and not significant increase of 14 % and 35 % for interstitial and perivascular fibrosis, respectively (Fig. 2A-C). Elevated perivascular CCL deposition was observed in untreated HT rats compared to NT rats, whereas proANP₃₁₋₆₇-treated HT rats showed similar perivascular CCL values to NT rats (Fig. 2A, D). Cardiac quantification of the pro-fibrotic transcription factor SMAD2 in hypertensive untreated rats revealed that nuclear phosphorylated SMAD2 had an average trend to increase of 28 ± 11.1% compared to NT rats (Fig. 2E). SMAD2 phosphorylation was significantly lower in hypertensive rats treated with proANP₃₁₋₆₇ compared to untreated rats.

### Cardiomyocyte Effects

Cardiomyocyte size was increased in untreated HT rats, whereas it was preserved in proANP₃₁₋₆₇-treated HT animals (Fig. 3A, B). In addition, t-tubule density was decreased in untreated HT hearts, whereas it was normal in proANP₃₁₋₆₇-treated animals (Fig. 3C). Similarly, the maximal intracellular distance from each point in the cytosol to the nearest t-tubule or surface membrane also increased in HT hearts, and this was prevented by proANP₃₁₋₆₇ treatment (Fig. 3D).

**Table 1: Haemodynamic, autoptic and echocardiographic characteristics of the study groups at 6 weeks.**

| | **NT** (n=7) | **HT** (n=7) | **HT + proANP₃₁₋₆₇** (n=15) |
|---|---|---|---|
| SBP (mmHg) | 163.9 ± 2.3 | *177.7* ± *5.6 ** | *179.3* ± *2.4 *** |
| DBP (mmHg) | 115.9 ± 3.6 | 121.2 ± 7.9 | 128.2 ± 2.3 |
| MAP (mmHg) | 131.6 ± 3.1 | 139.7 ± 6.8 | *144.9 ± 2.3 ** |
| BW (g) | 338.3 ± 1.7 | 335.6 ± 8.3 | 348.6 ± 4.4 |
| HW (mg) | 1258 ± 48.4 | *1519* ± *108.1 ** | 1365 ± 30.1 |
| HW/BW (mg/g) | 3.76 ± 0.1 | 4.62 ± *0.3* ***^{††}* | 3.97 ± 0.1 |
| EF (%) | 75.9 ± 4.1 | 82.6 ± 2.1 | 78.2 ± 1.9 |
| FS (%) | 46.5 ± 3.5 | 53.0 ± 2.3 | 48.7 ± 1.8 |
| E/A | 1.5 ± 0.1 | 1.1 ± *0.1 ^{††}* | 1.6 ± 0.1 |
| LA (mm) | 3.8 ± 0.1 | *4.9* ± *0.2 **^{††}* | *4.2* ± *0.1** |
| LV Mass (mg) | 823.3 ± 67.9 | 992.0 ± 43.4 * | 891.7 ± 12.9 |
| RWT | 0.49 ± 0.03 | *0.65* ± *0*.*03* ***^{††}* | 0.52 ± 0.02 |
| LV Vol;d (µl) | 240.8 ± 21.5 | 232.1 ± 13.8 | 265.1 ± 12.4 |
| LV Vol;s (µl) | 54.6 ± 4.6 | 41.7 ± 7.4 | 58.0 ± 6.3 |
| IVS;d (mm) | 1.7 ± 0.0 | *1.9* ± *0.0 *^{††}* | 1.7 ± 0.0 |
| IVS;s (mm) | 3.0 ± 0.0 | 3.3 ± 0.1 | 3.1 ± 0.0 |
| LVPW;d | 1.7 ± 0.1 | *2.2* ± *0.1* **^{†}* | 1.8 ± 0.0 |
| LVPW;s | 3.0 ± 0.1 | *3.4* ± *0.1* **^{††}* | 2.9 ± 0.1 |

### Legend

SBP = systolic blood pressure; DBP = diastolic blood pressure; MAP = mean arterial pressure; BW = body weight; HW = heart weight; EF = ejection fraction; FS = fractional shortening; E/A = ratio E wave to A wave; LA = left atrium diameter; LV Mass = left ventricular mass; RWT = relative wall thickness; LV Vol;d = left ventricular volume (diastole); LV Vol;s = left ventricular volume (systole); IVS;d = inter-ventricular septum (diastole); IVS;s = inter-ventricular septum (systole); LVPW;d = left ventricular posterior wall (diastole); and LVPW;s = left ventricular posterior wall (systole).
* p < 0.05 or ** p < 0.01 for difference from NT
† p < 0.05 or ††p < 0.01 for difference from HT + proANP₃₁₋₆₇

### In Vitro Effects on ANGII-Treated Cardiomyocytes

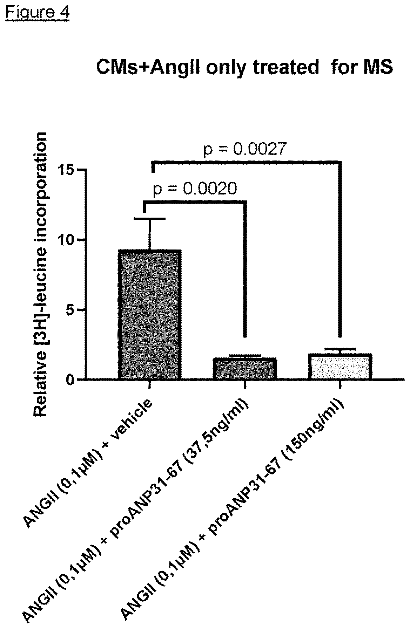

Neonatal cardiomyocytes were stimulated for 24h with the octapeptide ANGII in order to induce hypertrophy as indexed by [3H]-leucine incorporation. At the same time, cells were treated with either vehicle or with proANP₃₁₋₆₇ at two different concentrations: 37.5 ng/ml or 150 ng/ml. As shown in Figure 4, ANGII induced cardiac hypertrophy in vehicle treated cells, but the pathological hypertrophy was prevented by the two doses of proANP₃₁₋₆₇.

### Discussion

Administration of proANP₃₁₋₆₇ to HT rats at the dosages used in this example has a cardioprotective effect. The peptide was also shown to have protective effects on renal function and structure (data not shown). Notably, proANP₃₁₋₆₇ at the dosages used in this example did not reduce hypertension, indicating that these actions are independent of haemodynamic changes, indicating direct cardiorenal protective properties. While a direct effect of proANP₃₁₋₆₇ on the kidneys was previously known, a direct effect of the peptide on the heart has not previously been identified.

### Example 2: Effect of proANP₃₁₋₆₇ at 25 ng/kg/day on Hypertensive Rats

### Methods

Experiments were performed as described above in Example 1, using a group of 8 Dahl/SS rats which, at 7 weeks of age, were switched to a high salt diet as described above. After 2 weeks of high salt diet, this group was started on proANP₃₁₋₆₇ treatment. The rats were administered proANP₃₁₋₆₇ at a renal sub-therapeutic dosage of 25 ng/kg/day.

### Results

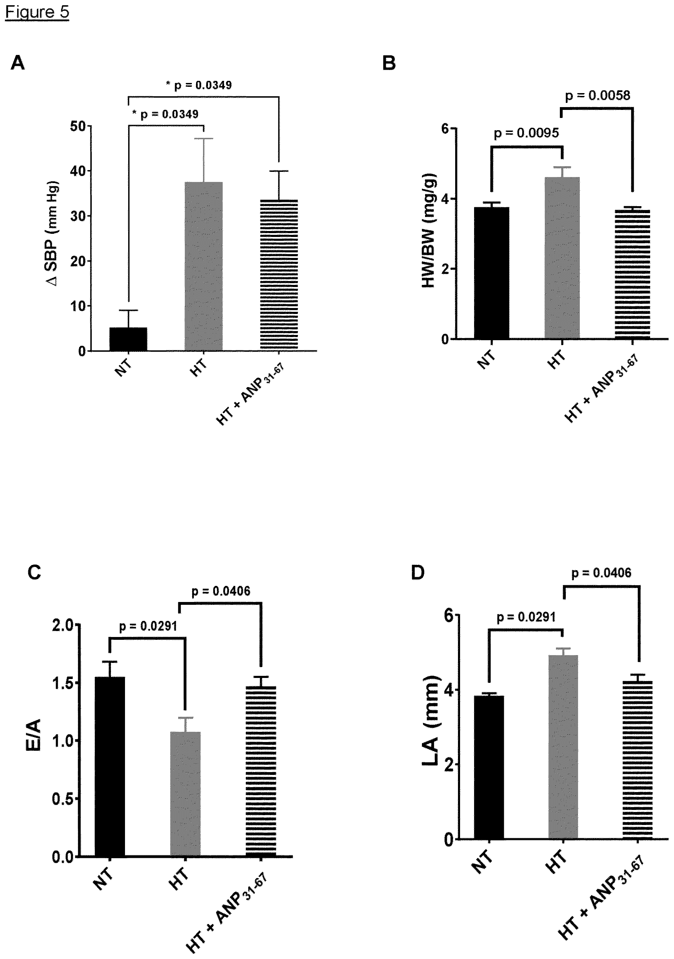

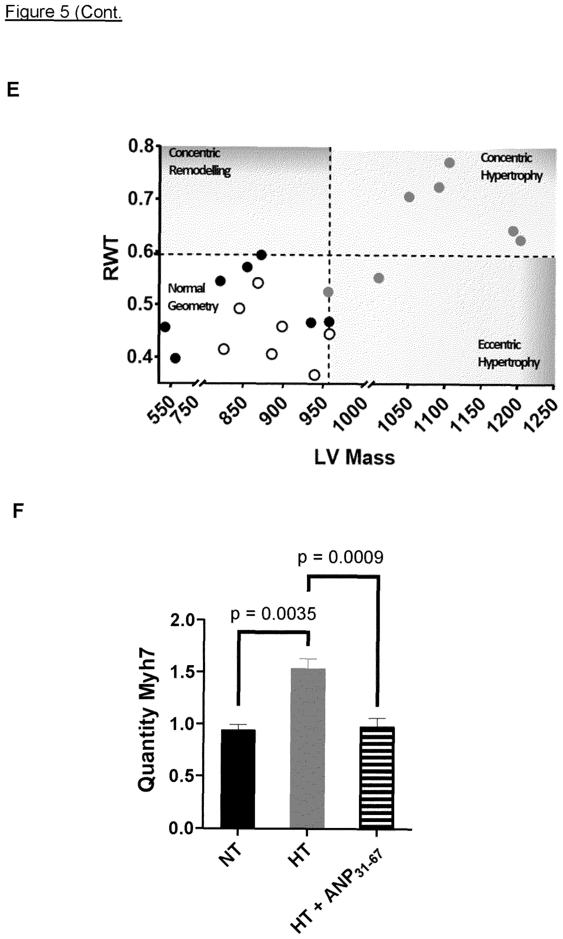

As for the higher doses of proANP₃₁₋₆₇ tested in Example 1, the dosage used in this example had no effect on systolic blood pressure (Table 2; Fig. 5A). As shown in Example 1, untreated HT rats demonstrated an increase in HW/BW relative to NT rats. Rats treated with the sub-therapeutic dose of 25 ng/kg/day proANP₃₁₋₆₇ demonstrated no increase in HW/BW (Table 2; Fig. 5B). These rats were also found to display a preserved diastolic function, as defined by E/A ratio (Table 2; Fig. 5C) and a much smaller change in LA diameter than the untreated HT group (Table 2; Fig. 5D). Whereas, as shown in Example 1, approximately 70 % untreated HT rats displayed concentric hypertrophy after receiving the high salt diet, all of the HT rats treated with proANP₃₁₋₆₇ displayed normal cardiac geometry (Table 2; Fig. 5E). ProANP₃₁₋₆₇ also attenuated the expression of the Myh7 (MHC-β) gene associated with adverse cardiac hypertrophy (Fig. 5F).

**Table 2: Haemodynamic, autoptic and echocardiographic characteristics of the study groups at 6 weeks.**

| | **NT** (n = 7) | **HT** (n = 7) | **HT + proANP₃₁₋₆₇** (n = 8) |
|---|---|---|---|
| SBP (mmHg) Hg) | 163.9 ± 2.3 | *177.7* ± *5.6* * | 176.5 ± 3.4 |
| DBP (mmHg) Hg) | 115.9 ± 3.6 | 121.2 ± 7.9 | 120.1 ± 4.7 |
| MAP (mmHg) | 131.6 ± 3.1 | 139.7 ± 6.8 | 138.6 ± 4.1 |
| BW (g) | 338.3 ± 1.7 | 335.6 ± 8.3 | 354.0 ± 8.3 |
| HW (mg) | 1258 ± 48.4 | *1519* ± *108.1 ^{†}* | 1304 ± 41.4 |
| HW / BW | 3.76 ± 0.1 | 4*.62 ± 0.3* * *^{††}* | 3.68 ± 0.07 |
| EF (%) | 75.9 ± 4.1 | 82.6 ± 2.1 | 79.6 ± 1.6 |
| FS (%) | 46.5 ± 3.5 | 53.0 ± 2.3 | 49.89 ± 1.7 |
| E/A | 1.49 ± 0.14 | *1.11* ± *0.09 ^{†}* | 1.47 ± 0.08 |
| LA (mm) | 3.65 ± 0.19 | *4.76* ± *0.18* * | 4.13 ± 0.17 |
| LV Mass (mg) | 823.3 ± 67.9 | *992.0* ± *43.4* * | 872.5 ± 22.5 |
| RWT | 0.49 ± 0.03 | *0.65* ± *0.03* ***^{††}* | 0.46 ± 0.02 |
| LV Vol;d (µl) | 240.8 ± 21.5 | 232.1 ± 13.8 | 288.5 ± 15.4 |
| LV Vol;s (µl) | 54.6 ± 4.6 | 41.7 ± 7.4 | 59.44 ± 6.2 |
| IVS;d (mm) | 1.73 ± 0.03 | *1.89* ± *0.04* * *^{††}* | 1.67 ± 0.03 |
| IVS;s (mm) | 3.05 ± 0.05 | 3.31 ± 0.13 | 3.12 ± 0.10 |
| LVPW;d (mm) | 1.75 ± 0.10 | *2.16* ± *0.08* ***^{††}* | 1.67 ± 0.04 |
| LVPW;s (mm) | 3.02 ± 0.07 | *3.39 ± 0.70* * *^{††}* | 2.81 ± 0.08 |

### Legend

SBP = systolic blood pressure; DBP = diastolic blood pressure; MAP = mean arterial pressure; BW = body weight; HW = heart weight; EF = ejection fraction; FS = fractional shortening; E/A = ratio E wave to A wave; LA = left atrium diameter; LV Mass = left ventricular mass; RWT = relative wall thickness; LV Vol;d = left ventricular volume (diastole); LV Vol;s = left ventricular volume (systole); IVS;d = inter-ventricular septum (diastole); IVS;s = inter-ventricular septum (systole); LVPW;d = left ventricular posterior wall (diastole); and LVPW;s = left ventricular posterior wall (systole).
* p < 0.05 or ** p < 0.01 for difference from NT
† p < 0.05 or †† p < 0.01 for difference from HT + proANP₃₁₋₆₇

### Discussion

The lower, 25 ng/kg/day dosage of proANP₃₁₋₆₇ was found not to display the protective effects on renal function seen for the higher dosages used in Example 1. However, the same cardioprotective effects were seen, demonstrating that the cardioprotective effects of proANP₃₁₋₆₇ are independent of its renal activity.

## Claims

1. A polypeptidic compound for use in prevention or treatment of cardiac remodelling in a subject, wherein said polypeptidic compound comprises the amino acid sequence set forth in SEQ ID NO: 1, or an amino acid sequence having at least 90 % sequence identity thereto, and wherein said compound comprises at least 34 amino acid residues and no more than 50 amino acid residues.

2. The polypeptidic compound for use according to claim 1, wherein said polypeptidic compound is the proANP₃₁₋₆₇ peptide, consisting of the amino acid sequence set forth in SEQ ID NO: 1.

3. The polypeptidic compound for use according to claim 1 or 2, wherein said cardiac remodelling is ventricular hypertrophy and/or ventricular fibrosis.

4. The polypeptidic compound for use according to any one of claims 1 to 3, wherein said therapy comprises:
(i) administering said polypeptidic compound subcutaneously to the subject at a dosage in the range of 25 to 2000 ng/kg/day; or
(ii) administering said polypeptidic compound to the subject intravenously at a dosage in the range of 5 to 1000 ng/kg/day; or
(iii) administering said polypeptidic compound to the subject intranasally at a dosage in the range of 5 to 1000 ng/kg/day.

5. The polypeptidic compound for use according to claim 4, wherein:
(i) in part (i) said dosage is in the range 25-95 ng/kg/day; or
(ii) in part (ii) said dosage is in the range 6-23 ng/kg/day; or
(iii) in part (iii) said dosage is in the range 8-30 ng/kg/day.

6. The polypeptidic compound for use according to any one of claims 1 to 5 wherein
the subject:
(i) does not have heart failure with a symptom severity level of NYHA Class III or Class IV; or
(ii) does not have overt symptoms of chronic congestive heart failure (CHF) or acute decompensated congestive heart failure (ADCHF).

7. The polypeptidic compound for use according to claim 6, wherein the subject does not have overt symptoms of heart failure and/or a cardiorenal syndrome.

8. The polypeptidic compound for use according to claim 7, wherein the subject is at risk of heart failure.

9. The polypeptidic compound for use according to claim 7 or 8, wherein said subject has hypertension.

10. The polypeptidic compound for use according to claim 9, wherein said subject has resistant hypertension.

11. The polypeptidic compound for use according to claim 7, wherein said subject has:
(i) an endocrine disorder, optionally wherein said endocrine disorder is diabetes;
(ii) a heart valve disorder;
(iii) myocarditis;
(iv) amyloidosis;
(v) cardiomyopathy;
(vi) an arrhythmia, optionally wherein said arrhythmia is tachycardia or bradycardia;
(vii) haemochromatosis;
(viii) chronic obstructive pulmonary disease;
(ix) sarcoidosis; or
(x) has had a heart attack;
or wherein said subject is undergoing cardiotoxic therapy, optionally wherein said cardiotoxic therapy is chemotherapy or radiotherapy.

12. The polypeptidic compound for use according to any one of claims 6 to 11, wherein further cardiac remodelling is prevented.

13. The polypeptidic compound for use according to any one of 6 to 12, wherein diastolic dysfunction is reduced or prevented.

14. The polypeptidic compound for use according to claim 12 or 13, wherein administration of said peptide to said subject reduces or prevents the development of heart failure.

15. The polypeptidic compound for use according to any one of claims 1 to 14 wherein said treatment or prevention does not also comprise administration of a diuretic agent to said subject.

## Patentansprüche

1. Polypeptidverbindung zur Verwendung in der Vorbeugung oder Behandlung von kardialem Remodeling bei einem Patienten, wobei die Polypeptidverbindung die in SEQ-ID Nr. 1 dargelegte Aminosäuresequenz oder eine Aminosäuresequenz umfasst, mit mindestens 90% Sequenzidentität zu dieser aufweist, und wobei die Verbindung mindestens 34 Aminosäurereste und nicht mehr als 50 Aminosäurereste umfasst.

2. Polypeptidverbindung zur Verwendung nach Anspruch 1, wobei die Polypeptidverbindung das proANP₃₁₋₆₇-Peptid ist, das aus der in SEQ-ID Nr. 1 dargelegten Aminosäuresequenz besteht.

3. Polypeptidverbindung zur Verwendung nach Anspruch 1 oder 2, wobei das kardiale Remodeling ventrikuläre Hypertrophie und/oder ventrikuläre Fibrose ist.

4. Polypeptidverbindung zur Verwendung nach einem der Ansprüche 1 bis 3, wobei die Therapie umfasst:
(i) subkutanes Verabreichen der Polypeptidverbindung an den Patienten in einer Dosierung im Bereich von 25 bis 2000 ng/kg/Tag; oder
(ii) intravenöses Verabreichen der Polypeptidverbindung an den Patienten in einer Dosierung im Bereich von 5 bis 1000 ng/kg/Tag; oder
(iii) intranasales Verabreichen der Polypeptidverbindung an den Patienten in einer Dosierung im Bereich von 5 bis 1000 ng/kg/Tag.

5. Polypeptidverbindung zur Verwendung nach Anspruch 4, wobei:
(i) in Teil (i) die Dosierung im Bereich von 25-95 ng/kg/Tag liegt; oder
(ii) in Teil (ii) die Dosierung im Bereich von 6-23 ng/kg/Tag liegt; oder
(iii) in Teil (iii) die Dosierung im Bereich von 8-30 ng/kg/Tag liegt.

6. Polypeptidverbindung zur Verwendung nach einem der Ansprüche 1 bis 5, wobei der Patient:
(i) keine Herzinsuffizienz mit einem Symptomschweregrad von NYHA-Klasse III oder Klasse IV aufweist; oder
(ii) keine offensichtlichen Symptome von chronischer Stauungsherzinsuffizienz (CHF) oder akuter dekompensierter Stauungsherzinsuffizienz (ADCHF) aufweist.

7. Polypeptidverbindung zur Verwendung nach Anspruch 6, wobei der Patient keine offensichtlichen Symptome von Herzinsuffizienz und/oder eines kardiorenalen Syndroms aufweist.

8. Polypeptidverbindung zur Verwendung nach Anspruch 7, wobei bei dem Patienten das Risiko von Herzinsuffizienz besteht.

9. Polypeptidverbindung zur Verwendung nach Anspruch 7 oder 8, wobei der Patient an Hypertonie leidet.

10. Polypeptidverbindung zur Verwendung nach Anspruch 9, wobei der Patient an therapieresistenter Hypertonie leidet.

11. Polypeptidverbindung zur Verwendung nach Anspruch 7, wobei der Patient aufweist:
(i) eine endokrine Störung, gegebenenfalls wobei die endokrine Störung Diabetes ist;
(ii) eine Herzklappenerkrankung;
(iii) Myokarditis;
(iv) Amyloidose;
(v) Kardiomyopathie;
(vi) eine Arrhythmie, gegebenenfalls wobei die Arrhythmie Tachykardie oder Bradykardie ist;
(vii) Hämochromatose;
(viii) chronisch obstruktive Lungenerkrankung;
(ix) Sarkoidose; oder
(x) einen Herzinfarkt hatte;
oder wobei sich der Patient einer kardiotoxischen Therapie unterzieht, gegebenenfalls wobei die kardiotoxische Therapie Chemotherapie oder Strahlentherapie ist.

12. Polypeptidverbindung zur Verwendung nach einem der Ansprüche 6 bis 11, wobei weiterem kardialen Remodeling vorgebeugt wird.

13. Polypeptidverbindung zur Verwendung nach einem der Ansprüche 6 bis 12, wobei diastolische Dysfunktion verringert oder vorgebeugt wird.

14. Polypeptidverbindung zur Verwendung nach Anspruch 12 oder 13, wobei Verabreichung des Peptids an den Patienten die Entwicklung von Herzinsuffizienz verringert oder vorbeugt.

15. Polypeptidverbindung zur Verwendung nach einem der Ansprüche 1 bis 14, wobei die Behandlung oder Vorbeugung nicht auch die Verabreichung eines Diuretikums an den Patienten umfasst.

## Revendications

1. Composé polypeptidique pour utilisation dans la prévention ou le traitement du remodelage cardiaque chez un sujet, dans lequel ledit composé polypeptidique comprend la séquence d'acides aminés présentée dans SEQ ID NO : 1, ou une séquence d'acides aminés présentant au moins 90 % d'identité de séquence avec celle-ci, et dans lequel ledit composé comprend au moins 34 résidus d'acides aminés et pas plus de 50 résidus d'acides aminés.

2. Composé polypeptidique pour utilisation selon la revendication 1, dans lequel ledit composé polypeptidique est le peptide proANP₃₁₋₆₇, consistant en la séquence d'acides aminés présentée dans SEQ ID NO : 1.

3. Composé polypeptidique pour utilisation selon la revendication 1 ou 2, dans lequel ledit remodelage cardiaque est une hypertrophie ventriculaire et/ou une fibrose ventriculaire.

4. Composé polypeptidique pour utilisation selon l'une quelconque des revendications 1 à 3, dans lequel ladite thérapie comprend :
(i) l'administration dudit composé polypeptidique par voie sous-cutanée au sujet à un dosage dans la plage de 25 à 2000 ng/kg/jour ; ou
(ii) l'administration dudit composé polypeptidique au sujet par voie intraveineuse à un dosage dans la plage de 5 à 1000 ng/kg/jour ; ou
(iii) l'administration dudit composé polypeptidique au sujet par voie intranasale à un dosage dans la plage de 5 à 1000 ng/kg/jour.

5. Composé polypeptidique pour utilisation selon la revendication 4, dans lequel :
(i) dans la partie (i) ledit dosage est dans la plage de 25 à 95 ng/kg/jour ; ou
(ii) dans la partie (ii) ledit dosage est dans la plage de 6 à 23 ng/kg/jour ; ou
(iii) dans la partie (iii) ledit dosage est dans la plage de 8 à 30 ng/kg/jour.

6. Composé polypeptidique pour utilisation selon l'une quelconque des revendications 1 à 5 dans lequel le sujet :
(i) ne souffre pas d'insuffisance cardiaque avec un niveau de gravité des symptômes de classe III ou IV de la NYHA ; ou
(ii) ne présente pas de symptômes manifestes d'insuffisance cardiaque congestive chronique (ICC) ou d'insuffisance cardiaque congestive aiguë décompensée (ADCHF).

7. Composé polypeptidique pour utilisation selon la revendication 6, dans lequel le sujet ne présente pas de symptômes manifestes d'insuffisance cardiaque et/ou un syndrome cardiorénal.

8. Composé polypeptidique pour utilisation selon la revendication 7, dans lequel le sujet présente un risque d'insuffisance cardiaque.

9. Composé polypeptidique pour utilisation selon la revendication 7 ou 8, dans lequel ledit sujet souffre d'hypertension.

10. Composé polypeptidique pour utilisation selon la revendication 9, dans lequel ledit sujet souffre d'une hypertension résistante.

11. Composé polypeptidique à utiliser selon la revendication 7, dans lequel ledit sujet présente :
(i) un trouble endocrinien, facultativement dans lequel ledit trouble endocrinien est le diabète ;
(ii) un trouble des valvules cardiaques ;
(iii) une myocardite ;
(iv) une amylose ;
(v) une cardiomyopathie ;
(vi) une arythmie, facultativement dans lequel ladite arythmie est une tachycardie ou une bradycardie ;
(vii) une hémochromatose ;
(viii) une maladie pulmonaire obstructive chronique ;
(ix) une sarcoïdose ; ou
(x) a eu une crise cardiaque ;
ou dans lequel ledit sujet subit une thérapie cardiotoxique, facultativement dans lequel ladite thérapie cardiotoxique est une chimiothérapie ou une radiothérapie.

12. Composé polypeptidique pour utilisation selon l'une quelconque des revendications 6 à 11, dans lequel un remodelage cardiaque ultérieur est empêché.

13. Composé polypeptidique pour utilisation selon l'une quelconque des revendications 6 à 12, dans lequel le dysfonctionnement diastolique est réduit ou empêché.

14. Composé polypeptidique pour utilisation selon la revendication 12 ou 13, dans lequel l'administration dudit peptide audit sujet réduit ou empêche le développement d'une insuffisance cardiaque.

15. Composé polypeptidique pour utilisation selon l'une quelconque des revendications 1 à 14, dans lequel ledit traitement ou ladite prévention ne comprend pas également l'administration d'un agent diurétique audit sujet.
